# EUROPÄISCHE PATENTSCHRIFT

(11) **EP 2 264 051 B1**
(45) Veröffentlichungstag und Bekanntmachung des Hinweises auf die Patenterteilung: **24.08.2016**
(21) Anmeldenummer: 10175016.4
(22) Anmeldetag: 11.06.2005
(51) Int. Cl.: C07K 14/32, C12P 13/14, C12R 1/10, C12N 5/10, C12N 15/52, C12P 13/04

(54) **Neue, Polyaminosäuren bildende oder abbauende Genprodukte von Bacillus licheniformis und darauf aufbauende verbesserte biotechnologische Produktionsverfahren**
New gene products of bacillus licheniformis forming or decomposing polyamino acids and associated improved biotechnological production method
Nouveaux produits génétiques formant ou recyclant des acides poly-aminés à partir de bacillus licheniformis et procédé de production biotechnologique amélioré basé sur ceux-ci

(30) Priorität: 26.06.2004 DE 102004030938
(43) Veröffentlichungstag der Anmeldung: 22.12.2010
(62) Teilanmeldung aus: 05750181.9
(73) Patentinhaber: BASF SE, 67056 Ludwigshafen am Rhein (DE)
(72) Erfinder: Feesche, Jörg, 40699, Erkrath (DE); Bessler, Cornelius, 40231, Düsseldorf (DE); Evers, Stefan, 40822, Mettmann (DE); Maurer, Karl-Heinz, 40699, Erkrath (DE); Ehrenreich, Armin, 37077, Göttingen (AT); Veith, Birgit, 40211, Düsseldorf (DE); Liesegang, Heiko, 37120, Bovenden (DE); Singer, Anke, 42699 Solingen (DE); Herzberg, Christina, 37434, Bilshausen (DE); Gottschalk, Gerhard, 37176, Nörten-Hardenberg (DE)

(56) Entgegenhaltungen:
- EP-A- 0 410 638
- EP-A2- 2 284 184
- EP-A2- 2 287 178
- WO-A-99/25864
- WO-A-02/055671
- WO-A2-02/29113
- VEITH B ET AL: "THE COMPLETE GENOME SEQUENCE OF BACILLUS LICHENIFORMIS DSM13, AN ORGANISM WITH GREAT INDUSTRIAL POTENTIAL" JOURNAL OF MOLECULAR MICROBIOLOGY AND BIOTECHNOLOGY, HORIZON SCIENTIFIC PRESS, WYMONDHAM,, GB, Bd. 7, Nr. 4, 2004, Seiten 204-211, XP009047713 ISSN: 1464-1801 -& DATABASE EMBL [Online] 21. September 2004 (2004-09-21), "Bacillus licheniformis DSM 13, complete genome" XP002339836 gefunden im EBI accession no. EM_PRO:AE017333 Database accession no. AE017333
- DATABASE Geneseq [Online] 13. August 2002 (2002-08-13), XP002339837 gefunden im EBI accession no. GSN:ABK75634 Database accession no. ABK75634
- DATABASE Geneseq [Online] 13. August 2002 (2002-08-13), XP002339838 gefunden im EBI accession no. GSN:ABK74415 Database accession no. ABK74415
- URUSHIBATA YUJI ET AL: "Characterization of the Bacillus subtilis ywsC gene, involved in gamma-polyglutamic acid production" JOURNAL OF BACTERIOLOGY, Bd. 184, Nr. 2, Januar 2002 (2002-01), Seiten 337-343, XP002339775 ISSN: 0021-9193 -& DATABASE EMBL [Online] 11. April 2001 (2001-04-11), "Bacillus subtilis ywsC, ywtA, ywtB, ywtC genes, complete cds." XP002339839 gefunden im EBI accession no. EM_PRO:AB046355 Database accession no. AB046355
- PATENT ABSTRACTS OF JAPAN Bd. 2000, Nr. 16, 8. Mai 2001 (2001-05-08) & JP 2001 017182 A (NAGASE & CO LTD), 23. Januar 2001 (2001-01-23) -& DATABASE EMBL [Online] 8. Februar 2002 (2002-02-08), "Process for producing poly-gamma-glutamic acid." XP002339840 gefunden im EBI accession no. EM_PRO:E50424 Database accession no. E50424 -& DATABASE JPO Proteins [Online] 31. Januar 2002 (2002-01-31), "Process for producing poly-gamma-glutamic acid." XP002339841 gefunden im EBI accession no. JPOP:E82283 Database accession no. E82283 -& DATABASE Geneseq [Online] 21. Juni 2001 (2001-06-21), "Bacillus subtilis IFO 3336 DNA encoding glutamate racemase enzyme." XP002339842 gefunden im EBI accession no. GSN:AAF82254 Database accession no. AAF82254
- LAPIDUS ALLA ET AL: "Co-linear scaffold of the Bacillus licheniformis and Bacillus subtilis genomes and its use to compare their competence genes." FEMS MICROBIOLOGY LETTERS. 19 MAR 2002, Bd. 209, Nr. 1, 19. März 2002 (2002-03-19) , Seiten 23-30, XP002339776 ISSN: 0378-1097
- DONG XU ET AL: "Phylogenetic relationships between Bacillus species and related genera inferred from comparison of 3' end 16S rDNA and 5' end 16S-23S ITS nucleotide sequences" INTERNATIONAL JOURNAL OF SYSTEMATIC AND EVOLUTIONARY MICROBIOLOGY, SOCIETY FOR GENERAL MICROBIOLOGY, READING,, GB, Bd. 53, Nr. 3, Mai 2003 (2003-05), Seiten 695-704, XP002903460 ISSN: 1466-5026
- BIRRER G A ET AL: "Gamma-poly(lutamic acid ) formation by Bacillus licheniformis 9945a: physiological and and biochemical studies" INTERNATIONAL JOURNAL OF BIOLOGICAL MACROMOLECULES, BUTTERWORTH & CO., GUILDFORD, GB, Bd. 16, Nr. 5, Oktober 1994 (1994-10), Seiten 265-275, XP002103917 ISSN: 0141-8130
- CROMWICK A M ET AL: "Effects of pH and aeration on gamma-poly(glutamic acid) formation by Bacillus licheniformis in controlled fermentor cultures" BIOTECHNOLOGY AND BIOENGINEERING. INCLUDING: SYMPOSIUM BIOTECHNOLOGY IN ENERGY PRODUCTION AND CONSERVATION, JOHN WILEY & SONS. NEW YORK, US, Bd. 50, 1996, Seiten 222-227, XP002103912 ISSN: 0006-3592
- TROY FREDERIC A: "Chemistry of biosynthesis of the poly(upsilon-D-glutamyl) capsule in bacillus lichiformis", JOURNAL OF BIOLOGICAL CHEMISTRY, AMERICAN SOCIETY FOR BIOCHEMISTRY AND MOLECULAR BIOLOGY, US, vol. 248, no. 1, 10 January 1973 (1973-01-10), pages 305-315, XP003028356, ISSN: 0021-9258

## Beschreibung

Die vorliegende Erfindung betrifft verbesserte biotechnologische Produktionsverfahren durch Mikroorganismen, die durch eine Inaktivierung eines neuen Gens und dessen Genprodukt von *Bacillus licheniformis* und hinreichend ähnlicher Gene und Proteine, die *in vivo* an der Bildung, der Modifizierung und/oder dem Abbau von Polyaminosäuren beteiligt sind und hierfür genutzt werden können, gekennzeichnet sind, sowie Mikroorganismen, bei denen das für ein an der Bildung von Poly-Gamma-Glutamat beteiligtes Genprodukt codierende Gen ywtB funktionell inaktiviert ist.

Die vorliegende Erfindung liegt auf dem Gebiet der Biotechnologie, insbesondere der Herstellung von Wertstoffen durch Fermentation von Mikroorganismen, die zur Bildung der interessierenden Wertstoffe in der Lage sind. Hierzu zählt beispielsweise die Herstellung niedermolekularer Verbindungen, etwa von Nahrungsmittelergänzungsstoffen oder pharmazeutisch relevanten Verbindungen, oder von Proteinen, für welche aufgrund ihrer Diversität wiederum ein großes technisches Einsatzgebiet besteht. Im ersten Fall werden die Stoffwechseleigenschaften der betreffenden Mikroorganismen zur Herstellung der Wertstoffe ausgenutzt und/oder verändert; im zweiten Fall werden Zellen eingesetzt, die die Gene der interessierenden Proteine exprimieren. In beiden Fällen handelt es sich zumeist also um gentechnisch veränderte Organismen (GVO).

Zur Fermentation von Mikroorganismen besteht ein reichhaltiger Stand der Technik, insbesondere auch im großtechnischen Maßstab; er reicht von der Optimierung der betreffenden Stämme hinsichtlich der Bildungsrate und der Nährstoffausnutzung über die technische Gestaltung der Fermenter bis hin zur Gewinnnung der Wertstoffe aus den betreffenden Zellen selbst und/oder dem Fermentationsmedium. Hierfür kommen sowohl genetische und mikrobiologische als auch verfahrenstechnische und biochemische Ansätze zu tragen. Ziel der vorliegenden Erfindung ist es, diesen Prozeß hinsichtlich einer häufigen, den eigentlichen Fermentationschritt beeinträchtigenden Eigenschaft der eingesetzten Mikroorganismen zu verbessern, und zwar auf der Ebene der genetischen Eigenschaften der eingesetzten Stämme.

Für die großtechnische, biotechnologische Produktion werden die betreffenden Mikroorganismen in Fermentern kultiviert, die ihren Stoffwechseleigenschaften entsprechend ausgelegt sind. Während der Kultivierung verstoffwechseln sie das angebotene Substrat und bilden neben dem eigentlichen Produkt üblicherweise eine Vielzahl weiterer Substanzen, an denen in der Regel kein Interesse besteht und/oder die - wie nachfolgend erläutert - zu Schwierigkeiten bei der Fermentation oder der Aufarbeitung führen können.

Fermentationen sind üblicherweise hochkomplexe Prozesse bei denen eine Vielzahl von verschiedenen Parametern eingestellt und überwacht werden muß. So handelt es sich beispielsweise sehr häufig um aerobe Prozesse, das heißt die eingesetzten Mikroorganismen müssen während der gesamten Dauer der Fermentation ausreichend mit Sauerstoff versorgt werden (Kontrolle der Begasungsrate). Weitere Beispiele für solche Parameter sind die Reaktorgeometrie, die sich forwährend verändernde Zusammensetzung des Nährmediums, der pH-Wert oder die CO₂-Bildungsrate. Ein besonders wichtiger Parameter, sowohl hinsichtlich der Wirtschaftlichkeit, als auch bezüglich der Prozeßführung an sich ist der notwendige Energieeintrag beispielsweise über Rührsysteme, die für eine möglichst vollständige Durchmischung des Reaktorinhalts sorgen. Darüber wird neben der Substratverteilung auch die ausreichende Versorgung der Organismen mit Sauerstoff sichergestellt.

Nach Beendigung der Fermentation müssen üblicherweise neben der Abtrennung der Produktionsorganismen eine Aufreinigung und/oder Aufkonzentrierung des interessierenden Wertstoffs aus dem sogenannten Fermenterbrei vorgenommen werden. Der Aufarbeitungsprozeß kann beispielsweise verschiedene Chromatographie- und/oder Filtrationsschritte aufweisen. Somit sind neben dem Gehalt an Wertstoffen auch die biophysikalischen Eigenschaften des Fermenterbreis, insbesondere seine Viskosität unmittelbar nach Beendigung der Fermentation für den Erfolg des gesamten Aufarbeitungsprozesses entscheidend.

Dessen Eigenschaften werden auch durch die Stoffwechselaktivitäten der gewählten Mikroorganismen beeinflußt, wobei auch unerwünschte Effekte auftreten können. Hierzu gehört beispielsweise eine häufig während der Fermentation zunehmende Viskosität des Nährmediums. Dies beeinträchtigt die Durchmischung und somit den Stofftransport und die Sauerstoffversorgung innerhalb des Reaktors. Zusätzliche Schwierigkeiten ergeben sich meist bei der anschließenden Aufarbeitung, weil erhöhte Viskositäten beispielsweise die Effizienz von Filtrationsprozessen erheblich beeinträchtigen.

Insbesondere von Spezies der Gattung *Bacillus* ist bekannt, daß sie Schleim bilden, welcher im wesentlichen aus poly-gamma-Glutamat (PGA) und/oder -Aspartat besteht, das heißt solchen Polyaminosäuren, die über die betreffenden gamma-Peptidbindungen verknüpft sind. In wissenschaftlichen Arbeiten an *Bacillus subtilis* werden hauptsächlich die drei Gene *ywsC, ywtA* und *ywtB* beziehungsweise die davon abgeleiteten Genprodukte mit der Bildung von poly-gamma-Glutamat in Verbindung gebracht; am Abbau ist das Genprodukt von *ywtD* beteiligt. Die allgemeine Genbezeichnung *"ywt"* ist dabei synonym mit den Abkürzungen *"cap"* und *"pgs",* die für dieselben Funktionen geläufig sind. Dies wird im folgenden dargestellt.

Die Publikation "Physiological and biochemical characteristics of poly gamma-glutamate synthetase complex of Bacillus subtilis" (2001) von M. Ashiuchi, et al., im Eur. J. Biochem., Band 268, Seiten 5321 - 5328, beschreibt den aus den drei Untereinheiten PgsB, PgsC und PgsA bestehenden Enzymkomplex PgsBCA (Poly-gamma-Glutamat-Synthetase-Komplex BCA) aus *B. subtilis.* Demnach handelt es sich bei diesem Komplex um eine untypische Amid-Ligase, die sowohl das D- als auch das L-Enantiomer von Glutamat zum entsprechenden Polymer umsetzt. Ein darin beschriebenes Gendisruptions-Experiment ist dieser Veröffentlichung zufolge als Beleg dafür anzusehen, daß dieser Komplex der einzige ist, der bei *B. subtilis* diese Reaktion katalysiert.

Y. Urushibata et al. belegen in der Publikation "Characterization of the Bacillus subtilis ywsC gene, involved in gamma-polyglutamic acid production" (2002), in J. Bacteriol., Band 184, Seiten 337-343, unter anderem über Deletionsmutationen an den drei Genen *ywsC, ywtA* und *ywtB,* daß die drei in *B. subtilis* für die Bildung von PGA verantwortlichen Genprodukte von diesen drei Genen codiert werden. Sie bilden in dieser Reihenfolge und zusammen mit dem nachfolgenden Gen ywtC, in diesem Mikroorganismus ein zusammenhängendes Operon.

Daß im Genom von *B. subtilis* stromabwärts von *ywtC* in einem eigenen Operon, ein weiteres, für den Stoffwechsel von PGA relevantes Gen liegt, zeigen T. Suzuki und Y. Tahara in der Publikation "Characterization of the Bacillus subtilis ywtD gene, whose product is involved in gamma-polyglutamic acid degradation" (2003), J. Bacteriol., Band 185, Seiten 2379 - 2382. Dieses Gen codiert für eine DL-Endopeptidase, die PGA zu hydrolysieren vermag und somit als gamma-DL-Glutamyl-Hydrolase bezeichnet werden kann.

Einen aktuellen Überblick über diese Enzyme liefert zusätzlich der Artikel "Biochemistry and molecular genetics of poly-gamma-glutamate synthesis" von M. Ashiuchi und H. Misono in Appl. Microbiol. Biotechnol., Band 59, Seiten 9 - 14 von 2002. Darin werden die zu *pgsB, pgsC* und *pgsA* homologen, für den PGA-Synthase-Komplex codierenden Gene in *B*. anthracis als *capB, capC* und *capA* bezeichnet. Das stromabwärts gelegene Gen wird diesem Artikel zufolge in *B. anthracis* als *dep* (für "D-PGA-Depolymerase") und in *B. subtilis* als *pgdS* (für "PGA-Depolymerase") bezeichnet.

Im heutigen Stand der Technik werden diese Enzymaktivitäten bereits positiv genutzt, hauptsächlich zur Herstellung von poly-gamma-Glutamat als Rohstoff, beispielsweise zum Einsatz in der Kosmetik, allerdings ohne daß bislang - insbesondere aus *B. licheniformis -* ihre genauen DNA- beziehungsweise Aminosäuresequenzen bekannt gewesen sind. So offenbart beispielsweise die Anmeldung JP 08308590 A die Herstellung von PGA durch Fermentation der PGAproduzierenden Stämme selbst, nämlich von *Bacillus*-Spezies wie *B. subtilis* und *B. licheniformis*; ferner wird darin die Gewinnung dieses Rohstoffs aus dem Kulturmedium beschrieben. *B. subtilis* var. chunkookjang stellt der Anmeldung WO 02/055671 A1 zufolge einen hierfür besonders geeigneten Mikroorganismus dar.

Somit besteht bei einigen Fermentationen ein Interesse an GLA, als dem durch die Fermentation zu produzierenden Wertstoff.

Bei allen anderen Fermentationen besteht jedoch das Interesse an der Herstellung anderer Wertstoffe; dabei bedeutet die Bildung von Polyaminosäuren aus den oben ausgeführten Gründen eine negative Begleiterscheinung. Eine typische Vorgehensweise, der auf deren Bildung zurückzuführenden erhöhten Viskosität des Fermentationsmediums Herr zu werden, ist die Erhöhung der Rührerdrehzahl. Dies wirkt sich jedoch auf den Energieeintrag aus. Zudem werden die fermentierten Mikroorganismen dadurch zunehmenden Scherkräften ausgesetzt, was einen erheblich Streßfaktor für sie darstellt. Sehr hohe Viskositäten, schließlich, können auch dadurch nicht überwunden werden, so daß ein vorzeitiger Abbruch der Fermention erforderlich sein kann, obgleich ansonsten die Produktion noch fortgesetzt werden könnte.

Schleimbildung, als negative Begleiterscheinung zahlreicher Fermentationsprozesse, kann sich somit aus vielerlei Gründen negativ auf das gesamte Fermentationsergebnis auswirken. Herkömmliche Methoden, laufende Fermentationen trotz einer zunehmenden Viskosität des Nährmediums erfolgreich fortzuführen, können nur als unzulänglich bezeichnet werden, insbesondere deshalb, weil sie keine ursächliche Bekämpfung darstellen.

Es stellte sich somit die vordringlichere Aufgabe, eine unerwünschte Bildung von Schleim, insbesondere eines auf Poly-gamma-Aminosäuren wie Poly-gamma-Glutamat zurückzuführenden Schleims während der Fermentation von Mikroorganismen möglichst weitgehend zu unterbinden. Insbesondere sollte eine Lösung gefunden werden, die eine ursächliche Bekämpfung darstellt. Einen weiteren Aspekt bildet die Bereitstellung der betreffenden Gene für eine positive Nutzung der GLA-synthetisierenden Genprodukte.

Eine Lösung dieser Aufgabe ist ein Verfahren zum Verringern von auf Poly-Gamma-Glutamat zurückzuführenden Schleim, gekennzeichnet durch das Unterbinden der Funktion des vom Gen *ywtB* codierten Proteins YwtB (CapA, PgsA) mit einer Aminosäuresequenz, die zu der in SEQ ID NO. 8 angegebenen Aminosäuresequenz mindestens 90% Identität aufweist, als einem an der Bildung von Polyaminosäuren beteiligten Enzym beziehungsweise als Untereinheit eines solchen Enzyms.

In einer Ausführungsform ist das Verfahren dadurch gekennzeichnet, dass die Funktion des Proteins YwtB während der Fermentation des Mikroorganismus unterbunden ist.

In einer weiteren Ausführungsform ist das Verfahren durch eine Verringerung des auf Polyaminosäuren zurückzuführenden Schleims auf 50% gekennzeichnet.

In einer weiteren Ausführungsform ist das Verfahren dadurch gekennzeichnet, dass der Mikroorganismus ein Bakterium ist, und/oder ein gram-negatives oder ein gram-positives Bakterium ist.

In einer weiteren Ausführungsform ist das Verfahren dadurch gekennzeichnet, dass der Mikroorganismus ausgewählt ist aus einer der Gattungen *Escherichia, Klebsiella, Pseudomonas, Xanthomonas, Bacillus, Staphylococcus* oder *Corynebacterium.*

In einer weiteren Ausführungsform ist das Verfahren durch folgende Schritte zum Unterbinden der Funktion des vom Gen *ywtB* codierten Proteins YwtB gekennzeichnet:
a) Auswählen zweier Bereiche der Sequenz SEQ ID NO. 7,
b) Klonieren der Bereiche in einen Vektor, so dass sie einen für ein nichtaktives Protein codierenden Teil flankieren, oder so, dass sie direkt aufeinanderfolgen unter Auslassung des dazwischenliegenden Bereichs,
c) Deletieren des Gens *ywtB* mit dem in Schritt b) hergestellten Vektor, und
d) Nachweisen der Gen-Deletion.

In einer weiteren Ausführungsform ist das Verfahren dadurch gekennzeichnet, dass die Funktion des vom Gen *ywtB* codierten Proteins YwtB (CapA, PsgA) durch Einsatz einer Nukleinsäure mit einer Deletions- oder Insertionsmutation, vorzugsweise umfassend die jeweils mindestens 70 bis 150 Nukleinsäurepositionen umfassenden Randsequenzen des für das Protein codierenden Bereichs, unterbunden ist.

Eine weitere Lösung ist ein Verfahren zur Herstellung eines Wertstoffes durch Fermentation eines Mikroorganismus, dadurch gekennzeichnet, dass während der Fermentation die Bildung von Poly-Gamma-Glutamat durch den Mikroorganismus durch Unterbinden der Funktion des vom Gen *ywtB* codierten Proteins YwtB (CapA, PgsA) mit einer Aminosäuresequenz, die zu der in SEQ ID NO. 8 angegebenen Aminosäuresequenz mindestens 90% Identität aufweist, als einem an der Bildung von Polyaminosäuren beteiligten Enzym beziehungsweise als Untereinheit eines solchen Enzyms, verringert ist.

In einer Ausführungsform ist das Verfahren dadurch gekennzeichnet, dass die Funktion des vom Gen *ywtB* codierten Proteins YwtB (CapA, PgsA) durch ein Verfahren mit folgenden Schritten unterbunden ist:
a) Auswählen zweier Bereiche der Sequenz SEQ ID NO. 7,
b) Klonieren der Bereiche in einen Vektor, so dass sie einen für ein nichtaktives Protein codierenden Teil flankieren, oder so, dass sie direkt aufeinanderfolgen unter Auslassung des dazwischenliegenden Bereichs,
c) Deletieren des Gens *ywtB* mit dem in Schritt b) hergestellten Vektor, und
d) Nachweisen der Gen-Deletion.

In einer weiteren Ausführungsform ist das Verfahren dadurch gekennzeichnet, dass die Funktion des vom Gen *ywtB* codierten Proteins YwtB (CapA, PsgA) durch Einsatz einer Nukleinsäure mit einer Deletions- oder Insertionsmutation, vorzugsweise umfassend die jeweils mindestens 70 bis 150 Nukleinsäurepositionen umfassenden Randsequenzen des für das Protein codierenden Bereichs, unterbunden ist.

In einer weiteren Ausführungsform ist das Verfahren dadurch gekennzeichnet, dass der Wertstoff ein Naturstoff, ein Nahrungsmittelergänzungsstoff oder eine pharmazeutisch relevante Verbindung oder ein Enzym ist.

In einer weiteren Ausführungsform ist das Verfahren dadurch gekennzeichnet, dass das Enzym ausgewählt ist aus der Gruppe der α-Amylasen, Proteasen, Cellulasen, Lipasen, Oxidoreduktasen, Peroxidasen, Laccasen, Oxidasen und Hemicellulasen.

Eine weitere Lösung ist die Verwendung einer Nukleinsäure codierend für ein an der Bildung von Poly-Gamma-Glutamat beteiligtes Protein YwtB (CapA, PgsA) mit einer Aminosäuresequenz, die zu der in SEQ ID NO. 8 angegebenen Aminosäuresequenz mindestens 90% Identität aufweist, oder jeweils Teilen davon zum Unterbinden der Funktion des Proteins YwtB (CapA, PgsA) als einem an der Bildung von Polyaminosäuren beteiligten Enzym beziehungsweise als Untereinheit eines solchen Enzyms.

Eine weitere Lösung ist die Verwendung einer Nukleinsäure codierend für ein an der Bildung von Poly-Gamma-Glutamat beteiligtes Protein YwtB (CapA, PgsA) mit einer Aminosäuresequenz, die zu der in SEQ ID NO. 8 angegebenen Aminosäuresequenz mindestens 90% Identität aufweist, oder jeweils Teilen davon zum Verringern des auf Poly-Gamma-Glutamat zurückzuführenden Schleims auf 50% während der Fermentation eines Mikroorganismus.

In einer Ausführungsform ist die Verwendung dadurch gekennzeichnet, dass die Nukleinsäure die Sequenz SEQ ID NO. 7 hat.

Eine weitere Lösung ist die Verwendung einer Nukleinsäure mit einer Deletions- oder Insertionsmutation umfassend die jeweils mindestens 70 bis 150 Nukleinsäurepositionen umfassenden Randsequenzen des für ein Protein YwtB (CapA, PsgA) mit der Aminosäuresequenz, die zu der in SEQ ID NO. 8 angegebenen Aminosäuresequenz mindestens 90% Identität aufweist, codierenden Bereichs zur Verringerung des auf Poly-Gamma-Glutamat zurückzuführenden Schleims während der Fermentation eines Mikroorganismus.

Eine weitere Lösung ist ein Mikroorganismus, bei dem das für ein an der Bildung von Poly-Gamma-Glutamat beteiligtes Genprodukt codierende Gen *ywtB* funktionell inaktiviert ist, wobei die codierende Nukleotidsequenz *ywtB* eine Nukleotidsequenz besitzt, die zu der in SEQ ID NO. 7 angegebenen Nukleotidsequenz mindestens 94% Identität aufweist.

In einer Ausführungsform ist der Mikroorganismus *Bacillus licheniformis.*

Weiterhin werden die zugehörige Nukleinsäure *ywtB* und darauf aufbauend die Verwendung der zugehörigen Nukleinsäuren zur Senkung der auf Polyaminosäuren zurückzuführenden Schleimbildung während der Fermentation des Mikroorganismus sowie entsprechende Fermentationsverfahren von Mikroorganismen offenbart Bei der erfindungsgemäßen Senkung der Schleim bildung auf genetischer Ebene ist das Gen *ywtB* funktionell inaktiviert. Hinzu kommt die positive Nutzung dieses Gens beziehungsweise der abgeleiteten Genprodukte zur Herstellung von Poly-gamma-Glutamat.

Diese prinzipiell auf alle fermentierbaren Mikroorganismen, insbesondere auf solche der Gattung *Bacillus* anwendbare Erfindung führt dazu, daß die für die fermentative Produktion von anderen Wertstoffen als Polyaminosäuren, insbesondere von pharmazeutisch relevanten niedermolekularen Verbindungen oder von Proteinen, eingesetzten Mikroorganismen auf genetischer Ebene daran gehindert werden, Polyaminosäuren, insbesondere GLA zu bilden. Dies wirkt sich zum einen vorteilhaft auf die Viskosität des Kulturmediums und darüber auf die Durchmischbarkeit, den Sauerstoffeintrag und die aufzuwendende Energie aus; zum anderen wird die Aufarbeitung des interessierenden Produkts erheblich erleichtet. Zudem wird ein Großteil der eingesetzten Rohstoffe, etwa der N-Quelle, nicht in ein nicht interessierendes Produkt umgewandelt, so daß insgesamt eine höhere Fermentationsausbeute zu erwarten ist.

Das genannte Gen kann für eine positive Nutzung des GLA-synthetisierenden Genprodukts verwendet werden, nämlich indem das abgeleitete Protein YwtB biotechnologisch gebildet und in den es produzierenden Zellen oder unabhängig davon als Katalysator in entsprechende Reaktionsansätze eingebracht wird.

Offenbart wird ein an der Bildung von Polyaminosäuren beteiligtes Protein YwtB (CapA, PgsA), das von einer Nukleotidsequenz *ywtB* codiert wird, die zu der in SEQ ID NO. 7 angegebenen Nukleotidsequenz mindestens 72% Identität aufweist.

Dieses spezielle Enzym wurde durch eine Analyse des Genoms von *B. licheniformis* DSM 13 erhalten (siehe Beispiel 1). Über die in der vorliegenden Anmeldung in SEQ ID NO. 7 und 8 angegebenen Nukleotid- und Aminosäuresequenzen wird dieses Protein nacharbeitbar zur Verfügung gestellt (siehe Beispiel 1).

Hierbei handelt es sich in Übereinstimmung mit den einleitend angeführten Literaturangaben um die dritte Untereinheit des Poly-gamma-Glutamat-Synthetase-Komplexes. Als das hierzu nächstähnliche, im Stand der Technik bekannte Protein wurde das Homologe YwsA aus *B. subtilis* ermittelt, das in der Datenbank GenBank (National Center for Biotechnology Information NCBI, National Institutes of Health, Bethesda, MD, USA) unter der Zugangsnummer AB046355.1 vermerkt ist und auf Nukleinsäureebene eine Homologie von 67,1% Identität besitzt; auf Aminosäureebene liegt die Übereinstimmung bei 65,8% Identität (siehe Beispiel 2). Diese signifikanten Übereinstimmungen lassen nicht nur auf dieselbe biochemische Funktion schließen, sondern auch darauf, daß innerhalb des beanspruchten Bereichs eine Vielzahl verwandter Proteine mit derselben Funktion liegt.

Diesem Protein YwtB sind folgende Varianten zuzuordnen:
- Jedes entsprechende Protein YwtB, das von einer Nukleotidsequenz codiert wird, die zu der in SEQ ID NO. 7 angegebenen Nukleotidsequenz zunehmend bevorzugt mindestens 75%, 80%, 85%, 90%, 92%, 94%, 96%, 97%, 98%, 99% und besonders bevorzugt 100% Identität aufweist.
- Jedes an der Bildung von Polyaminosäuren beteiligte Protein YwtB (CapA, PgsA) mit einer Aminosäuresequenz, die zu der in SEQ ID NO. 8 angegebenen Aminosäuresequenz mindestens 70% Identität, zunehmend bevorzugt mindestens 75%, 80%, 90%, 95%, 96%, 97%, 98%, 99% und besonders bevorzugt 100% Identität aufweist.

Am stärksten ist jeweils das konkrete aus *B. licheniformis* DSM13 erhaltene Protein bevorzugt, weil dies mit der vorliegenden Anmeldung konkret beschrieben und zu 100% nacharbeitbar zur Verfügung gestellt wird.

Jeweils bevorzugt ist hierunter jeweils solch ein an der Bildung oder dem Abbau von Polyaminosäuren beteiligtes, zuvor beschriebenes Protein, welches natürlicherweise von einem Mikroorganismus gebildet wird, vorzugsweise von einem Bakterium, besonders bevorzugt von einem grampositiven Bakterium, hierunter bevorzugt von einem der Gattung *Bacillus,* hierunter besonders bevorzugt von einem der Spezies *B. licheniformis* und hierunter ganz besonders bevorzugt von *B. licheniformis* DSM13.

Denn der Aufgabe entsprechend bestand Interesse daran, die Fermentation von Mikroorganismen zu verbessern, wofür häufig Bakterien und hierunter besonders grampositive verwendet werden, insbesondere solche, die wie *Bacillus* in der Lage sind, gebildete Wertstoffe und Proteine zu sekretieren. Zudem besteht hierfür ein reichhaltiger technischer Erfahrungsschatz. Zudem konnten wie erwähnt die im Sequenzprotokoll angegebenen Proteine für *B. licheniformis,* konkret *B. licheniformis* DSM13 nachgewiesen werden. Es ist zu erwarten, daß mit zunehmendem Verwandtschaftsgrad der betreffenden Organismen ein zunehmendes Maß an Übereinstimmung der Nukleotid- und Aminosäuresequenzen und somit deren Austauschbarkeit einhergeht.

Weiterhin betrifft die Offenbarung
Nukleinsäure n:
- Für ein an der Bildung von Polyaminosäuren beteiligtes Genprodukt codierende Nukleinsäure *ywtB* (*capA, pgsA*) mit einer Nukleotidsequenz, die zu der in SEQ ID NO. 7 angegebenen Nukleotidsequenz mindestens 72% Identität aufweist;
- eine entsprechende Nukleinsäure *ywtB* mit einer Nukleotidsequenz, die zu der in SEQ ID NO. 7 angegebenen Nukleotidsequenz zunehmend bevorzugt mindestens 75%, 80%, 85%, 90%, 92%, 94%, 96%, 97%, 98%, 99% und besonders bevorzugt 100% Identität aufweist.

Die hiermit zur Verfügung gestellten Nukleinsäuren können nach an sich bekannten molekularbiologischen Methoden zur Inaktivierung beziehungsweise Aktivitätsverstärkung der zugehörigen Proteine eingesetzt werden. So sind Inaktivierungen beispielsweise über entsprechende Deletionsvektoren (siehe unten) möglich; die Verstärkung der Aktivität erfolgt vorteilhafterweise über eine Überexpression, die mithilfe eines Expressionsvektors erzielbar ist (siehe unten).

Die in die jeweils angegebenen Homologiebereiche fallenden entsprechenden Gene können aus den interessierenden Organismen beispielsweise mithilfe von Sonden erhalten werden, die sich anhand der Sequenz 7 herstellen lassen. Diese vollständigen Gene können auch als Vorlage für die Erzeugung von PCR-Primern dienen, über die aus entsprechenden Gesamt-DNA-Präparationen die betreffenden Gene erschlossen werden können; diese wiederum liefern die zuvor beschriebenen Proteine. Hierbei ist die Erfolgsquote in der Regel umso größer, je näher der betreffende Stamm mit dem verwandt ist, der für die Konstruktion der Sonde oder der PCR-Primer gedient hat, im vorliegenden Fall also zu *B. licheniformis.*

Jeweils bevorzugt ist hierunter jeweils solch eine Nukleinsäure, die natürlicherweise in einem Mikroorganismus enthalten ist, vorzugsweise einem Bakterium, besonders bevorzugt einem grampositiven Bakterium, hierunter bevorzugt einem der Gattung *Bacillus,* hierunter besonders bevorzugt einem der Spezies *B. licheniformis* und hierunter ganz besonders bevorzugt *B*. *licheniformis* DSM13.

Denn wie oben ausgeführt worden ist, besteht ein besonderes Interesse daran, diese Gene für Fermentationen von derartigen Mikroorganismen auszunutzen. Zum anderen ist mit der vorliegenden Offenbarung auch die Möglichkeit verbunden, über die hier beschriebenen Gene und/oder Proteine den Stoffwechsel der Polyaminosäuren, insbesondere gamma-Glutaminsäure zumindest in Teilen nachzustellen, wenn diese synthetisiert, modifiziert und/oder abgebaut werden sollen. Hierfür steigt, insbesondere in entsprechenden transgenen Wirtszellen im allgemeinen die Erfolgsquote, je mehr die betreffenden Gene mit denen der natürlichen Zellen übereinstimmen.

Zudem lassen sich Alternativen der Gene und der Proteine leicht aus prinzipiell allen natürlichen Organismen isolieren.

Weiterhin werden Nukleinsäuren offenbart, die für ein oben beschriebenes Protein codieren.

So bestehen besonders zwischen entfernt verwandten Spezies Unterschiede hinsichtlich des Gebrauchs synonymer, für die jeweiligen Aminosäuren codierender Codons, worauf auch der Proteinbiosyntheseapparat ausgerichtet ist, etwa über die verfügbare Anzahl der passenden beladenen tRNAs. Die Übertragung eines der genannten Gene in eine weniger verwandte Spezies kann dann besonders erfolgreich beispielsweise zur Deletionsmutation oder zur Synthese des betreffenden Proteins genutzt werden, wenn sie hinsichtlich der Codons entsprechend optimiert ist. Hierdurch können prozentual auf der DNA-Ebene zunehmende Unterschiede eingeführt werden, die auf der Aminosäureebene jedoch ohne Folge bleiben.

Weiterhin werden Vektoren offenbart, die einen zuvor bezeichneten Nukleinsäurebereich enthalten.

Denn um mit den Nukleinsäuren umzugehen, und damit insbesondere die Produktion von Proteinen vorzubereiten, werden sie geeigneterweise in Vektoren ligiert. Solche Vektoren sowie die zugehörigen Arbeitsmethoden sind im Stand der Technik ausführlich beschrieben. Vektoren sind in großer Zahl und Variationsbreite, sowohl für die Klonierung als auch für die Expression kommerziell erhältlich. Dazu gehören beispielsweise Vektoren, die sich von bakteriellen Plasmiden, von Bacteriophagen oder von Viren ableiten, oder überwiegend synthetische Vektoren. Ferner werden sie nach der Art der Zelltypen, in denen sie sich zu etablieren vermögen, beispielsweise nach Vektoren für gramnegative, für grampositive Bakterien, für Hefen oder für höhere Eukaryonten unterschieden. Sie bilden geeignete Ausgangspunkte beispielsweise für molekularbiologische und biochemische Untersuchungen sowie für die Expression des betreffenden Gens oder zugehörigen Proteins. Insbesondere zur Herstellung von Konstrukten zur Deletion oder Verstärkung der Expression sind wie - wie aus dem hierfür einschlägigen Stand der Technik hervorgeht - praktisch unerläßlich.

Die Vektoren können Klonierungsvektoren sein.

Denn Klonierungsvektoren eignen sich neben der Lagerung, der biologischen Amplifizierung oder der Selektion des interessierenden Gens für dessen molekularbiologische Charakterisierung. Gleichzeitig stellen sie transportierbare und lagerfähige Formen der beanspruchten Nukleinsäuren dar und sind auch Ausgangspunkte für molekularbiologische Techniken, die nicht an Zellen gebunden sind, wie beispielsweise die PCR oder In-vitro-Mutagenese-Verfahren.

Vorzugsweise handelt es sich bei Vektoren um Expressionsvektoren.

Denn derartige Expressionsvektoren sind die Basis dafür, die entsprechenden Nukleinsäuren in biologischen Produktionssystemen zu realisieren und damit die zugehörigen Proteine zu produzieren. Bevorzugt sind Expressionsvektoren, die die zur Expression notwendigen genetischen Elemente tragen, beispielsweise den natürlichen, ursprünglich vor diesem Gen lokalisierten Promotor oder einen Promotor aus einem anderen Organismus. Diese Elemente können beispielsweise in Form einer sogenannten Expressionskassette angeordnet sein. Alternativ können einzelne oder alle Regulationselemente auch von der jeweiligen Wirtszelle bereitgestellt werden. Besonders bevorzugt sind die Expressionsvektoren hinsichtlich weiterer Eigenschaften, wie beispielsweise die optimale Kopienzahl, auf das gewählte Expressionssystem, insbesondere die Wirtszelle (siehe unten) abgestimmt.

Weiterhin werden Zellen offenbart, die nach gentechnischer Modifizierung eine der zuvor bezeichneten Nukleinsäuren enthalten.

Denn diese Zellen enthalten die genetische Information zur Synthese eines Proteins. Hierunter sind insbesondere diejenigen Zellen gemeint, die nach an sich bekannten Verfahren mit den Nukleinsäuren versehen worden sind, beziehungsweise die sich von solchen Zellen ableiten. Dafür werden geeigneterweise solche Wirtszellen ausgewählt, die sich vergleichsweise einfach kultivieren lassen und/oder hohe Produktausbeuten liefern.

Grundsätzlich müssen für Länder, in denen menschliche embryonale Stammzellen nicht unter Patentschutz gestellt werden dürfen, solche erfindungsgemäßen menschlichen embryonalen Stammzellen aus dem Schutzbereich ausgenommen werden.

Zellen ermöglichen beispielsweise die Amplifizierung der entsprechenden Gene, aber auch deren Mutagenese oder Transkription und Translation und letztlich die biotechnologische Produktion der betreffenden Proteine. Diese genetische Information kann entweder extrachromosomal als eigenes genetisches Element, das heißt bei Bakterien in plasmidaler Lokalisation vorliegen oder in ein Chromosom integriert sein. Die Wahl eines geeigneten Systems hängt von Fragestellungen, wie beispielsweise die Art und Dauer der Lagerung des Gens, beziehungsweise des Organismus oder die Art der Mutagenese oder Selektion ab.

Hierzu zählen insbesondere diejenigen Zellen, die das Gen *ywtB* über einen Vektor *in trans* enthalten und somit für entsprechende Deletionen genutzt werden können (siehe unten).

Bevorzugt ist in einer solchen Zelle die genannte Nukleinsäure Teil eines Vektors insbesondere eines zuvor beschriebenen Vektors.

Bevorzugt sind darunter Wirtszellen, bei denen es sich um Bakterien handelt.

Denn Bakterien zeichnen sich durch kurze Generationszeiten und geringe Ansprüche an die Kultivierungsbedingungen aus. Dadurch können kostengünstige Verfahren etabliert werden. Zudem verfügt man bei Bakterien in der Fermentationstechnik über einen reichhaltigen Erfahrungsschatz. Für eine spezielle Produktion können aus verschiedensten, im Einzelfall experimentell zu ermittelnden Gründen wie Nährstoffquellen, Produktbildungsrate, Zeitbedarf etc. gramnegative oder grampositive Bakterien geeignet sein.

Bevorzugt handelt es sich um ein gramnegatives Bakterium, insbesondere eines der Gattungen *Escherichia coli, Klebsiella, Pseudomonas* oder *Xanthomonas,* insbesondere um Stämme von E. *coli* K12, E. *coli* B oder *Klebsiella planticola,* und ganz besonders um Derivate der Stämme *Escherichia coli* BL21 (DE3), E. *coli* RV308, *E. coli* DH5α, E. *coli* JM109, E. *coli* XL-1 oder *Klebsiella planticola* (Rf).

Denn bei gramnegativen Bakterien, wie beispielsweise E. *coli,* wird eine Vielzahl von Proteinen in den periplasmatischen Raum sekretiert. Dies kann für spezielle Anwendungen vorteilhaft sein. In der Anmeldung WO 01/81597 A1 wird ein Verfahren offenbart, nach welchem erreicht wird, daß auch gramnegative Bakterien die exprimierten Proteine ausschleusen. Die als bevorzugt genannten gramnegativen Bakterien sind in der Regel leicht, das heißt kommerziell oder über öffentliche Stammsammlungen zugänglich und im Zusammenspiel mit ebenfalls in großer Zahl zur Verfügung stehenden übrigen Komponenten wie etwa Vektoren auf spezifische Herstellbedingungen hin optimierbar.

Nicht minder bevorzugt handelt es sich um ein grampositives Bakterium, insbesondere eines der Gattungen *Bacillus, Staphylococcus* oder *Corynebakterium,* ganz besonders der Species *Bacillus lentus, B. licheniformis, B.* amyloliquefaciens, *B. subtilis, B. globigii* oder *B. alcalophilus, Staphylococcus camosus* oder *Corynebacterium glutamicum,* und hierunter wiederum ganz besonders bevorzugt um ein Derivat von *B. licheniformis* DSM 13.

Denn grampositive Bakterien besitzen den gramnegativen gegenüber den grundsätzlichen Unterschied, sekretierte Proteine sogleich in das die Zellen umgebende Nährmedium abzugeben, aus welchem sich, wenn das gewünscht ist, die exprimierten Proteine direkt aus dem Nährmedium aufreinigen lassen. Zudem sind sie mit den meisten Herkunftsorganismen für technisch wichtige Enzyme verwandt oder identisch und bilden meist selbst vergleichbare Enzyme, so daß sie über eine ähnliche Codon-Usage verfügen und ihr Protein-Syntheseapparat naturgemäß entsprechend ausgerichtet ist. Ganz besonders bevorzugt sind Derivate von *B. licheniformis* DSM 13 deshalb, weil sie zum einen ebenfalls im Stand der Technik als biotechnologische Produktionsstämme weit verbreitet sind und weil zum anderen mit der vorliegenden Anmeldung exakt die Gene und Proteine aus *B. licheniformis* DSM 13 zur Verfügung gestellt werden, so daß die Realisierung der vorliegenden Erfindung am ehesten in solchen Stämmen erfolgreich sein sollte.

Weiterhin werden Verfahren zur Herstellung eines oben beschriebenen Genprodukts YwtB offenbart.

Dazu gehört jedes Verfahren zur Herstellung eines oben beschriebenen Proteins, beispielsweise chemische Syntheseverfahren. Demgegenüber sind jedoch alle im Stand der Technik etablierten, oben in einzelnen Aspekten bereits angesprochenen molekularbiologischen, mikrobiologischen, beziehungsweise biotechnologischen Herstellverfahren bevorzugt. Deren Ziel besteht in erster Linie darin, die Proteine zu erhalten, um sie entsprechenden Anwendungen zur Verfügung zu stellen, beispielsweise für die Synthese von Poly-gamma-Glutamat.

Vorzugsweise handelt es sich dabei um Verfahren, die unter Einsatz einer oben bezeichneten Nukleinsäuren erfolgen, vorzugsweise unter Einsatz eines zuvor bezeichneten Vektors und besonders bevorzugt unter Einsatz einer zuvor bezeichneten Zelle erfolgen.

Denn durch die genannten Nukleinsäuren, insbesondere die im Sequenzprotokoll unter SEQ ID NO. 7 angegebene Nukleinsäure wird die entsprechend bevorzugte genetische Information in mikrobiologisch verwertbarer Form, das heißt für gentechnische Produktionsverfahren zur Verfügung gestellt. Zunehmend bevorzugt ist die Bereitstellung auf einem von der Wirtszelle besonders erfolgreich verwertbaren Vektor beziehungsweise von solchen Zellen selbst. Die betreffenden Produktionsverfahren sind dem Fachmann an sich bekannt.

Grundlage der zugehörigen Nukteinsauresequenzen konnen auch zellfreie Expressionssysteme sein, bei denen die Proteinbiosynthese *in vitro* nachvollzogen wird. Alle bereits oben ausgeführten Elemente können auch zu neuen Verfahren kombiniert werden, um Proteine herzustellen. Es ist dabei für jedes Protein eine Vielzahl von Kombinationsmöglichkeiten an Verfahrensschritten denkbar, so daß optimale Verfahren für jeden konkreten Einzelfall experimentell ermittelt werden müssen.

Weiterhin bevorzugt sind solche derartigen Verfahren, bei denen die Nukleotidsequenz in einem oder vorzugsweise mehreren Codons an die Codon-Usage des Wirtsstamms angepaßt worden ist.

Denn entsprechend dem oben Gesagten kann die Übertragung eines der genannten Gene in eine weniger verwandte Spezies dann besonders erfolgreich zur Synthese des betreffenden Proteins genutzt werden, wenn sie hinsichtlich des Gebrauchs der Codons entsprechend optimiert ist.

Weiterhin wird die Verwendung einer oben beschriebenen Nukleinsäure *ywtB* oder einer entsprechenden Nukleinsäure, die für eines der oben beschriebenen Proteine codiert oder jeweils Teilen davon, zur funktionellen Inaktivierung des jeweils zugehörigen Gens *ywtB* in einem Mikroorganismus offenbart.

Unter der funktionellen Inaktivierung ist im Sinne der vorliegenden Anmeldung jede Art von Modifikation oder Mutation zu verstehen, wonach die Funktion des betreffenden Proteins als ein an der Bildung von Polyaminosäuren beteiligtes Enzym beziehungsweise als Untereinheit eines solchen Enzyms unterbunden wird. Dazu gehört die Ausführungsform, daß ein praktisch vollständiges, aber inaktives Protein gebildet wird, daß inaktive Teile eines solchen Proteins in der Zelle vorliegen, bis hin zu den Möglichkeiten, daß das zugehörige Gen nicht mehr translatiert wird oder sogar vollständig deletiert ist. Somit besteht eine spezielle "Verwendung" dieser Faktoren beziehungsweise Gene dieser Ausführungsform nach darin, daß sie in der betreffenden Zelle eben nicht mehr auf ihre natürliche Weise zur Wirkung kommen. Dies wird diesem Erfindungsgegenstand zufolge auf genetischer Ebene dadurch erreicht, daß das betreffende Gen ausgeschaltet wird.

In bevorzugten Ausführungsformen handelt es sich bei beiden Verwendungen um solche, bei denen die funktionelle Inaktivierung beziehungsweise Aktivitätserhöhung während der Fermentation des Mikroorganismus erfolgt, vorzugsweise mit einer Verringerung des auf Polyaminosäuren zurückzuführenden Schleims auf 50%, besonders bevorzugt auf weniger als 20%, ganz besonders bevorzugt auf weniger als 5%, wobei wiederum alle dazwischenliegenden ganzzahligen oder gebrochenen Prozentwerte in entsprechend bevorzugter Abstufung zu verstehen sind.

Zur Bestimmung dieser Werte werden Zellen eines nichtbehandelten Stamms und eines behandelten Stamms unter ansonsten identischen Bedingungen fermentiert und geeigneterweise während der Fermentation die Viskosität des jeweiligen Mediums bestimmt. Da die Stämme ansonsten identisch sind, sind die Viskositätsunterschiede auf die unterschiedlichen Gehalte an Polyaminosäuren zurückzuführen. Dabei ist erfindungsgemäß jede Viskositätserniedrigung erwünscht. Prozentual vergleichbare Werte erhält man, indem man aus beiden Fermentationen Proben nimmt und nach an sich bekannten Methoden den Gehalt an Polyaminosäure-haltigem Schleim bestimmt. Zunehmend bevorzugt ist es, wenn der in der erfindungsgemäßen Probe bestimmbare Wert beim Übergang in die stationäre Wachstumsphase weniger als 50%, 40%, 30%, 20%, 10%, 5% und ganz besonders weniger als 1% des entsprechenden Werts der Vergleichsfermantation beträgt.

Für die Verwendung zur funktionellen Inaktivierung des Gens *ywtB* kann eine für ein nichtaktives Protein codierende Nukleinsäure mit einer Punktmutation eingesetzt. werden.

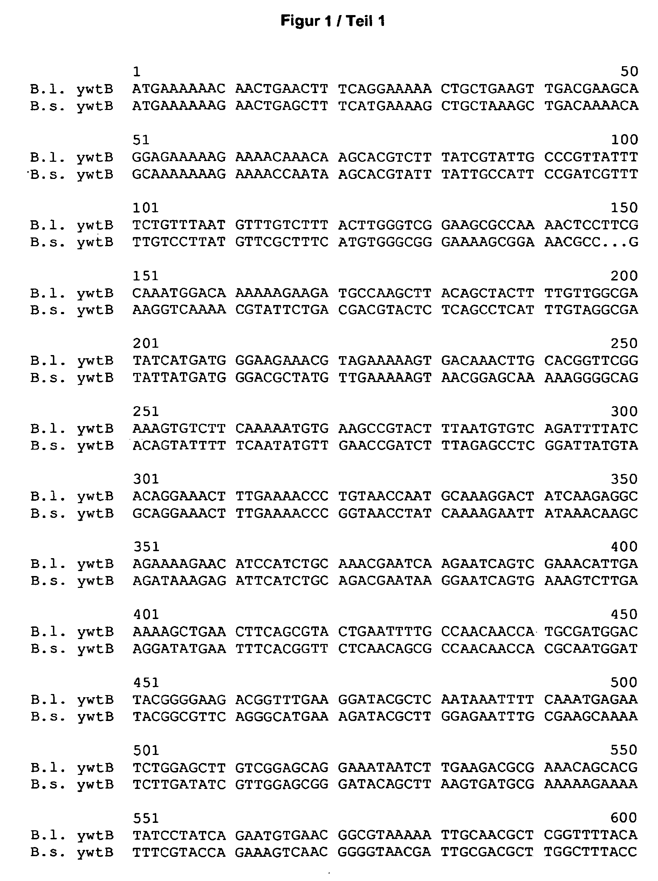

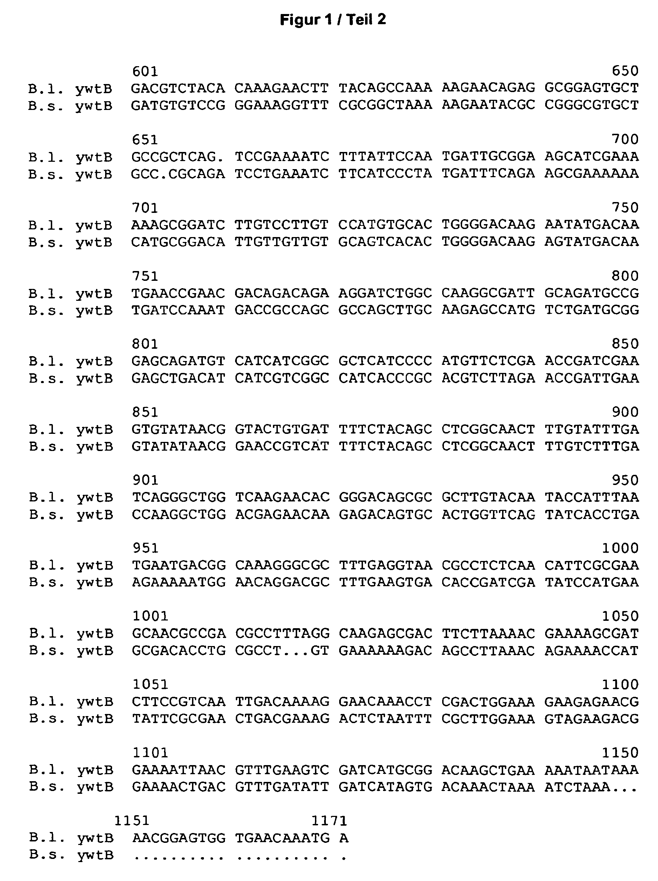

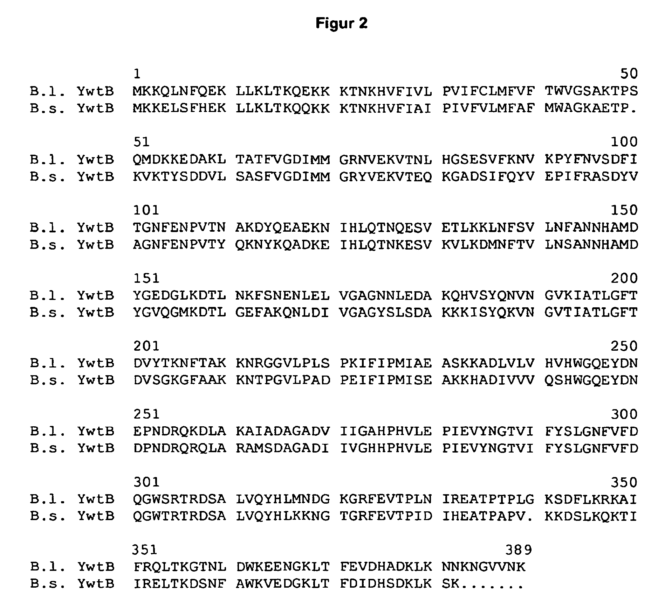

Derartige Nukleinsäuren können über an sich bekannte Verfahren zur Punktmutagenese erzeugt werden. Solche sind beispielsweise in einschlägigen Handbüchern wie dem von Fritsch, Sambrook und Maniatis, "Molecular cloning: a laboratory manual", Cold Spring Harbour Laboratory Press, New York, 1989, dargestellt. Zudem stehen hierfür inzwischen zahlreiche kommerzielle Baukästen zur Verfügung, etwa das QuickChange^{®}-Kit der Firma Stratagene, La Jolla, USA. Dessen Prinzip besteht darin, daß Oligonukleotide mit einzelnen Austauschen (Mismatch-Primer) synthetisiert und mit dem einzelsträngig vorgelegten Gen hybridisiert werden; anschließende DNA-Polymerisation ergibt dann entsprechende Punktmutanten. Hierfür können die jeweiligen Spezies-eigenen Sequenzen dieser Gene verwendet werden. Aufgrund der hohen Homologien ist es möglich und erfindungsgemäß besonders vorteilhaft, diese Reaktion anhand der mit SEQ ID NO. 7 zur Verfügung gestellten Sequenz durchzuführen. Diese Sequenz kann auch dazu dienen, entsprechende Mismatch-Primer für verwandte Spezies zu entwerfen, insbesondere anhand der in den Alignments der Figuren 1 und 2 identifizierbaren konservierten Bereiche.

Nach einer Ausführungsform dieser Verwendung wird für die funktionelle Inaktivierung jeweils eine Nukleinsäure mit einer Deletions- oder Insertionsmutation eingesetzt, vorzugsweise umfassend die jeweils mindestens 70 bis 150 Nukleinsäurepositionen umfassenden Randsequenzen des für das Protein codierenden Bereichs.

Auch diese Verfahren sind dem Fachmann an sich vertraut. Somit ist es möglich, die Bildung eines Faktors YwtB durch die Wirtszelle dadurch zu verhindern, daß ein Teil des betreffenden Gens auf einem entsprechenden Transformationsvektor über Restriktionsendonukleasen herausgeschnitten und der Vektor anschließend in den interessierenden Wirt transformiert wird, wo über die - bis dahin noch mögliche - homologe Rekombination das aktive Gen gegen die inaktive Kopie ausgetauscht wird. In der Ausführungsform der Insertionsmutation kann lediglich das intakte Gen unterbrechend oder anstelle eines Genteils ein anderes Gen, beispielsweise ein Selektionsmarker eingefügt werden. Hierüber ist das Mutationsereignis in an sich bekannter Weise phänotypisch überprüfbar.

Um diese jeweils notwendigen Rekombinationsereignisse zwischen dem in die Zelle eingeführten defekten Gen und der beispielsweise auf dem Chromosom endogen vorhandenen intakten Genkopie zu ermöglichen, ist nach dem derzeitigen Wissensstand eine Übereinstimmung in jeweils mindestens 70 bis 150 zusammenhängenden Nukleinsäurepositionen, jeweils in den beiden Randsequenzen zu dem nichtübereinstimmenden Teil nötig, wobei es auf den dazwischenliegenden Teil nicht ankommt. Dementsprechend sind solche Ausführungsformen bevorzugt, die lediglich zwei flankierende Regionen mit mindestens diesen Größen umfassen.

Für die Verwendung können Nukleinsäuren mit insgesamt zwei Nukleinsäureabschnitten eingesetzt werden, die jeweils mindestens 70 bis 150 Nukleinsäurepositionen umfassen und damit den für das Protein codierenden Bereich zumindest teilweise, vorzugsweise vollständig flankieren. Die flankierenden Bereiche können dabei ausgehend von den bekannten Sequenzen über an sich bekannte Methoden, beispielsweise mithilfe nach außen gerichteter PCR-Primer und einer Präparation genomischer DNA als Matrize ermittelt werden (anchored PCR). Denn allein um den Austausch der beiden Genkopien über homologe Rekombination zu ermöglichen, braucht es sich dabei nicht zwangsläufig um proteincodierende Abschnitte zu handeln. Der vorliegenden Erfindung zufolge können die hierfür benötigten Primer anhand der SEQ ID NO. 7 auch für andere Spezies grampositiver Bakterien und hierunter insbesondere für solche der Gattung *Bacillus* entworfen werden. Alternativ zu diesem experimentellen Ansatz können derartige, wenigstens zum Teil nichtcodierende Bereiche für viele dieser Gene aus verwandten Spezies, beispielsweise aus *B. subtilis* Datenbankeinträgen entnommen werden, beispielsweise der Datenbank SubtiList des Institute Pasteur, Paris, Frankreich (http://genolist.pasteur.fr/SubtiList/genome.cgi).

Die vorliegende Erfindung wird auch in der Form gentechnisch modifizierter Mikroorganismen verwirklicht, auf die das oben Gesagte entsprechend zutrifft.

Ganz allgemein sind das Mikroorganismen, bei denen das Gen *ywtB* funktionell inaktiviert ist.

Vorzugsweise sind dies Mikroorganismen, bei denen es sich um Bakterien handelt.

Hierunter sind solche Mikroorganismen bevorzugt, bei denen es sich um gramnegative Bakterien handelt, insbesondere solche der Gattungen *Escherichia coli, Klebsiella, Pseudomonas* oder *Xanthomonas,* insbesondere um Stämme von *E. coli* K12, *E. coli* B oder *Klebsiella planticola,* und ganz besonders um Derivate der Stämme *Escherichia coli* BL21 (DE3), *E. coli* RV308, *E. coli* DH5α, *E. coli* JM109, *E. coli* XL-1 oder *Klebsiella planticola* (Rf).

Mikroorganismen bei denen es sich um grampositive Bakterien handelt, insbesondere solche der Gattungen *Bacillus, Staphylococcus* oder *Corynebacterium,* ganz besonders der Species *Bacillus lentus, B. licheniformis, B. amyloliquefaciens, B. subtilis, B. globigii* oder *B. alcalophilus, Staphylococcus camosus* oder *Corynebacterium glutamicum* und hierunter ganz besonders um *B. licheniformis* DSM 13, sind nicht minder bevorzugt.

Der der vorliegenden Anmeldung zugrunde gelegenen Aufgabe zufolge sollten in erster Linie technische Fermentationsverfahren verbessert werden. Dementsprechend wird die Erfindung insbesondere in entsprechenden, erfindungsgemäßen Fermentationsverfahren verwirklicht.

Dabei handelt es sich ganz allgemein um Verfahren zur Fermentation eines zuvor beschriebenen, erfindungsgemäßen Mikroorganismus.

Den bisherigen Ausführungen entsprechend sind die hierdurch gekennzeichneten Verfahren entsprechend bevorzugt. Dazu gehört insbesondere , das Gen *ywtB* funktionell zu inaktivieren. Besonders bevorzugt wird hierfür auf die oben beschriebenen Nukleinsäuren zurückgegriffen, vor allem die unter SEQ ID NO. 7 angegebene. Dies gilt entsprechend auch für die für die jeweilige Fermentation als geeignet ausgewählten Spezies. Entsprechend dem oben Gesagten sind hierunter diejenigen zunehmend bevorzugt, die zu *B. licheniformis* DSM13 ein zunehmendes Maß an Verwandtschaft aufweisen, weil hiermit die Erfolgsaussichten beim Einsatz der angegebenen Nukleinsäuren steigen.

Unter den erfindungsgemäßen Fermentationsverfahren sind diejenigen zur Herstellung eines Wertstoffs bevorzugt, insbesondere zur Herstellung einer niedermolekularen Verbindung oder eines Proteins.

Denn dies ist das wichtigste Anwendungsgebiet für großtechnische Fermentationen.

Vorzugsweise sind das Verfahren, wobei es sich bei der niedermolekularen Verbindung um einen Naturstoff, einen Nahrungsmittelergänzungsstoff oder um eine pharmazeutisch relevante Verbindung handelt.

Auf diese Weise werden beispielsweise Aminosäuren oder Vitamine produziert, die besonders als Nahrungsmittelergänzungsstoffe Verwendung finden. Bei pharmazeutisch relevanten Verbindungen kann es sich um Vor- oder Zwischenstufen zu Medikamenten oder sogar um diese selbst handeln. In all diesen Fällen spricht man auch von Biotransformation, wonach die Stoffwechseleigenschaften der Mikroorganismen ausgenutzt werden, um die ansonsten aufwendige chemische Synthese ganz oder zumindest in einzelnen Schritten zu ersetzen.

Nicht minder bevorzugt sind entsprechende Verfahren, bei denen es sich bei dem auf diese Weise gebildeten Protein um ein Enzym handelt, insbesondere eines aus der Gruppe der α-Amylasen, Proteasen, Cellulasen, Lipasen, Oxidoreduktasen, Peroxidasen, Laccasen, Oxidasen und Hemicellulasen.

Industrielle Enzyme, die mit derartigen Verfahren hergestellt werden, finden beispielsweise in der Nahrungsmittelindustrie Verwendung. So dienen α-Amylasen beispielsweise dazu, um das Altbackenwerden von Brot zu verhindern oder um Fruchtsäfte zu klären. Proteasen werden zum Aufschluß von Proteinen verwendet. All diese Enzyme sind für den Einsatz in Wasch- und Reinigungsmitteln beschrieben, wobei insbesondere die von grampositiven Bakterien bereits natürlicherweise hergestellten *Subtili*sin-Proteasen einen prominenten Platz einnehmen. Insbesondere in der Textil- und Lederindustrie dienen sie der Aufarbeitung der natürlichen Rohstoffe. Ferner können all diese Enzyme wiederum im Sinne der Biotransformation als Katalysatoren für chemische Reaktionen eingesetzt werden.

Viele dieser Enzyme stammen ursprünglich aus *Bacillus*-Spezies und werden deshalb besonders erfolgreich in grampositiven Organismen, insbesondere solchen der Gattung *Bacillus* produziert, worunter in vielen Fällen auch Derivate von *B. licheniformis* DSM13 fallen. Insbesondere Produktionsverfahren, die auf diesen mikrobiellen Systemen beruhen, können mithilfe der vorliegenden Erfindung verbessert werden, weil insbesondere die in SEQ ID NO. 7 angegebene Sequenz aus eben diesem Organismus stammt.

Schließlich können die mit der vorliegenden Anmeldung zur Verfügung gestellten Faktoren auch positiv, das heißt im Sinne ihrer natürlichen Funktion eingesetzt werden, das heißt im Zusammenhang mit einer gezielten Herstellung von Poly-gamma-Glutamat.

Offenbart werden somit mikrobielle Verfahren zur Herstellung von Poly-gamma-Glutamat, bei dem eine der oben beschriebenen Nukleinsäuren *ywtB* oder eine entsprechende Nukleinsäure, die für ein oben beschriebenes Protein codiert, transgen zum Einsatz kommt, vorzugsweise unter Bildung des entsprechenden oben beschriebenen Proteins.

Hierunter sind Verfahren bevorzugt, bei denen ein Mikroorganismus der Gattung *Bacillus,* insbesondere *B. subtilis* oder *B. licheniformis* zum Einsatz kommt.

So ist es, wie beispielsweise in den Anmeldungen JP 08308590 A oder WO 02/055671 A1 beschrieben, möglich, GLA mikrobiell, und zwar in *B. subtilis* und *B. licheniformis* zu produzieren. Die mit der vorliegenden Anmeldung zur Verfügung gestellten DNA-Sequenzen können beispielsweise dazu genutzt werden, um in entsprechenden Zellen, die jeweiligen Genaktivitäten zu erhöhen und somit die Ausbeute zu steigern.

Alternativ hierzu sind nun auch zellfreie Verfahren zur Herstellung von Poly-gamma-Glutamat unter Beteiligung eines oben beschriebenen, an der Bildung von Polyaminosäuren beteiligten Genprodukts YwtB möglich, vorzugsweise unter Einsatz einer entsprechenden oben beschriebenen Nukleinsäure.

So können diese Faktoren beispielsweise in einem Bioreaktor zur Reaktion gebracht werden. Die Konstruktion solcher Enzym-Bioreaktoren ist an sich aus dem Stand der Technik bekannt.

Die nachfolgenden Beispiele erläutern die vorliegende Erfindung weiter.

### Beispiele

Alle molekularbiologischen Arbeitsschritte folgen Standardmethoden, wie sie beispielsweise in dem Handbuch von Fritsch, Sambrook und Maniatis "Molecular cloning: a laboratory manual", Cold Spring Harbour Laboratory Press, New York, 1989, oder vergleichbaren einschlägigen Werken angegeben sind. Enzyme, Baukästen (Kits) und Geräte wurden nach den Angaben der jeweiligen Hersteller eingesetzt.

### Beispiel 1

### Identifizierung der Gene ywsC, ywsC', ywtA, ywtB und ywtD aus B. lichenifonnis DSM 13

Aus dem von der Deutschen Sammlung von Mikroorganismen und Zellkulturen GmbH, Mascheroder Weg 1b, 38124 Braunschweig (http://www.dsmz.de) für jedermann erhältlichen Stamm *B. licheniformis* DSM 13 wurde nach Standardmethoden die genomische DNA präpariert, mechanisch fraktioniert und über Elektrophorese in einem 0,8%igen Agarosegel aufgetrennt. Für eine Schrotschußklonierung der kleineren Fragmente wurden die 2 bis 2,5 kb großen Fragmente aus dem Agarosegel eluiert, dephosphoryliert und als stumpf endende (blunt ended) Fragmente in die Smal-Restriktionsschnittstelle des Vektors pTZ19R-Cm ligiert. Dabei handelt es sich um ein Chloramphenicol-Resistenz verleihendes Derivat des von der Firma Fermentas (St. Leon-Rot) kommerziell erhältlichen Plasmids pTZ19R. Dadurch wurde eine Genbank der kleineren Fragmente erhalten. Als zweite Schrotschußklonierung wurden die durch eine partielle Restriktion mit dem Enzym Saulllal erhaltenen genomischen Fragmente in das SuperCos-1-Vektorsystem ("Cosmid Vector Kit") der Firma Stratagene, La Jolla, USA, ligiert, wodurch eine Genbank über die überwiegend größeren Fragmente erhalten wurde.

Aus den durch Transformation mit den betreffenden Genbanken erhältlichen Bakterien E. coli DH5α (D.Hannahan (1983): "Studies on transformation on Escherichia coli"; J. Mol. Microbiol., Band 166, Seiten 557 - 580) wurden die betreffenden rekombinanten Plasmide isoliert und sequenziert. Hierbei kam die Farbstoffabbruchmethode (dye terminator chemistry) zum Einsatz, durchgeführt durch die automatischen Sequenziergeräte Mega-BACE 1000/4000 (Fa. Amersham Bioscence, Piscataway, USA) und ABI Prism 377 (Fa. Applied Biosystems, Foster City, USA).

Auf diese Weise wurden unter anderem die im Sequenzprotokoll der vorliegenden Anmeldung angegebenen Sequenzen SEQ ID NO. 1, 3, 5, 7 und 9 erhalten, die in dieser Reihenfolge für die Gene *ywsC, ywsC'* (als verkürzte Variante von *ywsC*)*, ywtA, ywtB* und *ywtD* stehen. Die hiervon abgeleiteten Aminosäuresequenzen sind in entsprechender Reihenfolge in SEQ ID NO. 2,4, 6, 8 beziehungsweise 10 angegeben. Für das Gen beziehungsweise Protein *ywsC* (beziehungsweise YwsC) wird eine verkürzte Variante *ywsC'* (beziehungsweise YwsC') angegeben, weil der in Figur 6 gezeigte Vergleich der Aminosäuresequenzen für das homologe Protein in *B. subtilis* ein Polypeptid zeigt, das bei ansonsten recht hoher Homologie und deshalb vergleichbarer Aktivität N-terminal um 16 Aminosäuren kürzer ist.

### Reproduzierbarkeit

Diese Gene und Genprodukte können nun nach an sich bekannten Methoden, und ohne daß man die geschilderte Sequenzierung nacharbeiten muß, gezielt anhand dieser Sequenzen künstlich synthetisiert werden. Als weitere Alternative hierzu ist es möglich, die betreffenden Gene aus einem *Bacillus-Stamm,* insbesondere dem von der DSMZ erhältlichen Stamm *B. licheniformis* DSM 13, über PCR zu gewinnen, wobei die im Sequenzprotokoll angebenen jeweiligen Randsequenzen für die Synthese von Primern verwendet werden können. Bei Verwendung anderer Stämme werden die jeweils homologen Gene hierzu erhalten, wobei die PCR umso erfolgreicher sein sollte, je enger die ausgewählten Stämme mit *B. licheniformis* DSM 13 verwandt sind, weil damit eine zunehmende Sequenzübereinstimmung auch innerhalb der Primer-Bindungsregionen einhergehen dürfte.

### Beispiel 2

### Sequenzhomologien

Nach Ermittlung der DNA- und Aminosäuresequenzen gemäß Beispiel 1 wurden durch Recherche in den Datenbanken GenBank (National Center for Biotechnology Information NCBI, National Institutes of Health, Bethesda, MD, USA; http://www.ncbi.nlm.nih.gov) und *Subtilist* des Institute Pasteur, Paris, Frankreich (http://genolist.pasteur.fr/*Subtilist*/genome.cgi) die jeweils nächstähnlichen, bisher bekannten Homologe ermittelt.

Die ermittelten DNA- beziehungsweise Aminosäuresequenzen wurden über die in den Figuren 1 bis 10 dargestellten Alignments einander gegenübergestellt; hierfür wurde das Computerprogramm Vector NTI^{®} Suite Version 7, verwendet, welches von der Firma Informax Inc., Bethesda, USA, erhältlich ist. Hierbei wurden die Standard-Parameter dieses Programms angewendet, das heißt für den Vergleich der DNA-Sequenzen: K-tuple size: 2; Number of best Diagonals: 4; Window size: 4; Gap penalty: 5; Gap opening penalty: 15 und Gap extension penalty: 6,66. Für den Vergleich der Aminosäure-Sequenzen galten folgende Standard-Parameter: K-tuple size: 1; Number of best Diagonals: 5; Window size: 5; Gap penalty: 3; Gap opening penalty: 10 und Gap extension penalty: 0,1. Die Ergebnisse dieser Sequenzvergleiche sind in folgender Tabelle 1 zusammengestellt, wobei als Zugangsnummem diejenigen aus der NCBI-Datenbank angegeben sind.

**Tabelle 1: Nächstähnliche Gene beziehungsweise Proteine zu den in Beispiel 1 ermittelten Genen und Proteinen.**

| in B. *licheniformis* gefundenes Gen oder Protein / SEQ ID NO. | nächstverwandtes Gen beziehungsweise Protein | Datenbankeintrag des nächsten verwandten Gens beziehungsweise Proteins | Homologie in % Identität |
|---|---|---|---|
| *ywsC* / 1 | *ywsC* aus *B. subtilis* | AB046355.1 | 75,4 |
| *ywsC' l* 3 | *ywsC* aus *B. subtilis* | AB046355.1 | 78,5 |
| *ywtA l* 5 | ywsA aus *B. subtilis* | AB046355.1 | 77,8 |
| *YwtB* / 7 | ywsB aus *B. subtilis* | AB046355.1 | 67,1 |
| *ywtD* / 9 | *ywtD* aus *B. subtilis* | AB080748 | 62,3 |
| YwsC / 2 | YwsC aus *B. subtilis* | AB046355.1 | 86,1 |
| YwsC' / 4 | YwsC aus *B. subtilis* | AB046355.1 | 89,6 |
| YwtA / 6 | YwsA aus *B. subtilis* | AB046355.1 | 89,9 |
| YwtB / 8 | YwsB aus *B. subtilis* | AB046355.1 | 65,8 |
| YwtD / 10 | YwsD aus *B. subtilis* | AB080748 | 57,3 |

### Beispiel 3

### Funktionelle Inaktivierung eines oder mehrerer der Gene ywsC, ywsC', ywtA und ywtB in B. licheniformis

### Prinzip der Herstellung eines Deletionsvektors

Jedes dieser Gene kann beispielsweise mittels eines sogenannten Deletionsvektors funktionell inaktiviert werden. Dieses Vorgehens ist an sich beispielsweise von J. Vehmaanperä et al. (1991) in der Publikation "Genetic manipulation of Bacillus amyloliquefaciens"; J. Biotechnol., Band 19, Seiten 221 - 240 beschrieben.

Ein geeigneter Vektor hierfür ist pE194, der in der Publikation "Replication and incompatibility properties of plasmid pE194 in Bacillus subtilis" von T.J. Gryczan et al. (1982), J. Bacteriol., Band 152, Seiten 722 - 735 charakterisiert ist. Der Vorteil dieses Deletionsvektors besteht darin, daß er einen Temperatur-abhängigen Replikationsursprung besitzt. Bei 33°C kann pE194 in der transformierten Zelle replizieren, so daß bei dieser Temperatur zunächst auf eine erfolgreiche Transformation selektiert wird. Anschließend werden die Zellen, die den Vektor enthalten, bei 42°C inkubiert. Bei dieser Temperatur repliziert der Deletionsvektor nicht mehr, und es wird ein Selektionsdruck auf die Integration des Plasmids über einen zuvor ausgewählten homologe Bereich in das Chromosom ausgeübt. Eine zweite homologe Rekombination über einen zweiten homologen Bereich führt dann zur Excision des Vektors zusammen mit der intakten Genkopie aus dem Chromosom und damit zur Deletion des *in vivo* chromosomal lokalisierten Gens. Möglich wäre auch als zweite Rekombination die Umkehrreaktion zur Integration, das heißt ein Herausrekombinieren des Vektors aus dem Chromosom, so daß das chromosomale Gen intakt bliebe. Die Gen-Deletion muß daher nach an sich bekannten Methoden, etwa im Southern-Blot nach Restriktion der chromosomalen DNA mit geeigneten Enzymen oder mit Hilfe der PCR-Technik anhand der Größe des amplifizierten Bereichs nachgewiesen werden.

Erforderlich ist also die Auswahl zweier homologer Bereiche des zu deletierenden Gens, die jeweils mindestens je 70 Basenpaare umfassen sollten, beispielsweise der 5'- und der 3'-Bereich des ausgewählten Gens. Diese werden so in den Vektor kloniert, daß sie einen für ein nichtaktives Protein codierenden Teil flankieren oder unter Auslassung des dazwischenliegenden Bereichs direkt aufeinanderfolgen. Hierdurch wird der Deletionsvektor erhalten.

### Deletion der hier betrachteten Gene ywsC, ywsC', ywtA und ywtB

Zur Konstruktion eines erfindungsgemäßen Deletionsvektors werden die 5'- und 3'-Bereiche eines dieser vier beziehungsweise drei Gene mittels PCR amplfiziert. Zur Konstruktion geeigneter Primer stehen die im Sequenzprotokoll angegebenen Sequenzen SEQ ID NO. 1, 3, 5 und 7 zur Verfügung, die aus B. *licheniformis* stammen, aufgrund zu erwartender Homologien aber auch für andere Spezies, insbesondere der Gattung *Bacillus* geeignet sein sollten.

Die beiden amplifizierten Bereiche werden geeigneterweise unmittelbar hintereinander auf einem für diese Arbeiten gebräuchlichen Vektor zwischenkloniert, zum Beispiel auf dem Vektor pUC18, der sich für Klonierungssschritte in E. coli. eignet.

Im nächsten Schritt erfolgt eine Umklonierung in den zur Deletion ausgewählten Vektor pE194 und dessen Transformation in *B. subtilis* DB104, etwa nach der Methode der ProtoplastenTransformation nach Chang & Cohen (1979; "High Frequency Transformation of Bacillus subtilis Protoplasts by Plasmid DNA"; Molec. Gen. Genet. (1979), Band 168, Seiten 111-115). Alle Arbeitsschritte müssen bei 33°C durchgeführt werden, um eine Replikation des Vektors zu gewährleisten.

In einem nächsten Schritt wird der zwischenklonierte Vektor ebenfalls mittels der Methode der Protoplastentransformation in den gewünschten Wirtsstamm, hier *B. licheniformis,* transformiert. Die solcherart erhaltenen und mit üblichen Methoden (Selektion über den Resistenzmarker des Plasmids; Kontrolle über Plasmidpräparation und PCR für das Insert) als positiv identifizierten Transformanten werden anschließend bei 42°C unter Selektionsdruck durch Zugabe von Erythromycin auf Anwesenheit des Plasmids kultiviert. Bei dieser Temperatur kann der Deletionsvektor nicht mehr replizieren, und es überleben nur solche Zellen, bei denen der Vektor in das Chromosom integriert ist, wobei diese Integration mit höchster Wahrscheinlichkeit in homologen oder identischen Bereichen stattfindet. Durch Kultivierung bei 33°C ohne Erythromycin-Selektionsdruck kann dann nachfolgend die Excision des Deletionsvektors induziert werden, wobei das chromosomal codierte Gen vollständig aus dem Chromosom entfernt wird. Der Erfolg der Deletion wird anschließend über Southern-Blot nach Restriktion der chromosomalen DNA mit geeigneten Enzymen oder mit Hilfe der PCR-Technik überprüft.

Solche Transformanten, bei denen das betreffende Gen deletiert ist, zeichnen sich zudem durch eine Einschränkung oder sogar die völlige Unfähigkeit zur Bildung der GLA aus.

### Beschreibung der Figuren

- Figur 1:: Alignment des Gens *ywtB* (SEQ ID NO. 7) aus *B. licheniformis* DSM 13 (B.I. ywtB) mit dem homologen Gen *ywtB* aus *B. subtilis* (B.s. ywtB).
- Figur 2:: Alignment des Proteins YwtB (SEQ ID NO. 8) aus *B. licheniformis* DSM 13 (B.I. YwtB) mit dem homologen Protein YwtB aus *B. subtilis* (B.s. YwtB).

## Patentansprüche

1. Verfahren zum Verringern von auf Poly-Gamma-Glutamat zurückzuführenden Schleim, **gekennzeichnet durch** das Unterbinden der Funktion des vom Gen *ywtB* codierten Proteins YwtB (CapA, PgsA) mit einer Aminosäuresequenz, die zu der in SEQ ID NO. 8 angegebenen Aminosäuresequenz mindestens 90% Identität aufweist, als einem an der Bildung von Polyaminosäuren beteiligten Enzym beziehungsweise als Untereinheit eines solchen Enzyms.

2. Verfahren gemäß Anspruch 1, **dadurch gekennzeichnet, dass** die Funktion des Proteins YwtB während der Fermentation des Mikroorganismus unterbunden ist.

3. Verfahren gemäß Anspruch 1 oder 2, **gekennzeichnet durch** eine Verringerung des auf Polyaminosäuren zurückzuführenden Schleims auf 50%.

4. Verfahren gemäß einem der Ansprüche 1, 2 oder 3, **dadurch gekennzeichnet, dass** der Mikroorganismus
a) ein Bakterium ist, und/oder
b) ein gram-negatives Bakterium ist, oder
c) ein gram-positives Bakterium ist.

5. Verfahren gemäß Anspruch 4, **dadurch gekennzeichnet, dass** der Mikroorganismus ausgewählt ist aus einer der Gattungen *Escherichia, Klebsiella, Pseudomonas, Xanthomonas, Bacillus, Staphylococcus* oder *Corynebacterium.*

6. Verfahren gemäß einem der vorherigen Ansprüche, **gekennzeichnet durch** folgende Schritte zum Unterbinden der Funktion des vom Gen *ywtB* codierten Proteins YwtB:
a) Auswählen zweier Bereiche der Sequenz SEQ ID NO. 7,
b) Klonieren der Bereiche in einen Vektor, so dass sie einen für ein nichtaktives Protein codierenden Teil flankieren, oder so, dass sie direkt aufeinanderfolgen unter Auslassung des dazwischenliegenden Bereichs,
c) Deletieren des Gens *ywtB* mit dem in Schritt b) hergestellten Vektor, und
d) Nachweisen der Gen-Deletion.

7. Verfahren gemäß einem der vorherigen Ansprüche, **dadurch gekennzeichnet, dass** die Funktion des vom Gen *ywtB* codierten Proteins YwtB (CapA, PsgA) unterbunden ist durch Einsatz einer Nukleinsäure mit einer Deletions- oder Insertionsmutation, vorzugsweise umfassend die jeweils mindestens 70 bis 150 Nukleinsäurepositionen umfassenden Randsequenzen des für das Protein codierenden Bereichs.

8. Verfahren zur Herstellung eines Wertstoffes durch Fermentation eines Mikroorganismus, **dadurch gekennzeichnet, dass** während der Fermentation die Bildung von Poly-Gamma-Glutamat durch den Mikroorganismus verringert ist durch Unterbinden der Funktion des vom Gen *ywtB* codierten Proteins YwtB (CapA, PgsA) mit einer Aminosäuresequenz, die zu der in SEQ ID NO. 8 angegebenen Aminosäuresequenz mindestens 90% Identität aufweist, als einem an der Bildung von Polyaminosäuren beteiligten Enzym beziehungsweise als Untereinheit eines solchen Enzyms.

9. Verfahren gemäß Anspruch 8, **dadurch gekennzeichnet, dass** die Funktion des vom Gen *ywtB* codierten Proteins YwtB (CapA, PgsA) unterbunden ist durch ein Verfahren mit folgenden Schritten:
a) Auswählen zweier Bereiche der Sequenz SEQ ID NO. 7,
b) Klonieren der Bereiche in einen Vektor, so dass sie einen für ein nichtaktives Protein codierenden Teil flankieren, oder so, dass sie direkt aufeinanderfolgen unter Auslassung des dazwischenliegenden Bereichs,
c) Deletieren des Gens *ywtB* mit dem in Schritt b) hergestellten Vektor, und
d) Nachweisen der Gen-Deletion.

10. Verfahren gemäß Anspruch 8 oder 9, **dadurch gekennzeichnet, dass** die Funktion des vom Gen *ywtB* codierten Proteins YwtB (CapA, PsgA) unterbunden ist durch Einsatz einer Nukleinsäure mit einer Deletions- oder Insertionsmutation, vorzugsweise umfassend die jeweils mindestens 70 bis 150 Nukleinsäurepositionen umfassenden Randsequenzen des für das Protein codierenden Bereichs.

11. Verfahren gemäß Anspruch 8, 9 oder 10, **dadurch gekennzeichnet, dass** der Wertstoffen Naturstoff, ein Nahrungsmittelergänzungsstoff oder eine pharmazeutisch relevante Verbindung oder ein Enzym ist.

12. Verfahren gemäß Anspruch 11, **dadurch gekennzeichnet, dass** das Enzym ausgewählt ist aus der Gruppe der α-Amylasen, Proteasen, Cellulasen, Lipasen, Oxidoreduktasen, Peroxidasen, Laccasen, Oxidasen und Hemicellulasen.

13. Verwendung einer Nukleinsäure codierend für ein an der Bildung von Poly-Gamma-Glutamat beteiligtes Protein YwtB (CapA, PgsA) mit einer Aminosäuresequenz, die zu der in SEQ ID NO. 8 angegebenen Aminosäuresequenz mindestens 90% Identität aufweist, oder jeweils Teilen davon zum Unterbinden der Funktion des Proteins YwtB (CapA, PgsA) als einem an der Bildung von Polyaminosäuren beteiligten Enzym beziehungsweise als Untereinheit eines solchen Enzyms.

14. Verwendung einer Nukleinsäure codierend für ein an der Bildung von Poly-Gamma-Glutamat beteiligtes Protein YwtB (CapA, PgsA) mit einer Aminosäuresequenz, die zu der in SEQ ID NO. 8 angegebenen Aminosäuresequenz mindestens 90% Identität aufweist, oder jeweils Teilen davon zum Verringern des auf Poly-Gamma-Glutamat zurückzuführenden Schleims auf 50% während der Fermentation eines Mikroorganismus.

15. Verwendung gemäß Anspruch 13 oder 14, **dadurch gekennzeichnet, dass** die Nukleinsäure die Sequenz SEQ ID NO. 7 hat.

16. Verwendung einer Nukleinsäure mit einer Deletions- oder Insertionsmutation umfassend die jeweils mindestens 70 bis 150 Nukleinsäurepositionen umfassenden Randsequenzen des für ein Protein YwtB (CapA, PsgA) mit der Aminosäuresequenz, die zu der in SEQ ID NO. 8 angegebenen Aminosäuresequenz mindestens 90% Identität aufweist, codierenden Bereichs zur Verringerung des auf Poly-Gamma-Glutamat zurückzuführenden Schleims während der Fermentation eines Mikroorganismus.

17. Mikroorganismus, bei dem das für ein an der Bildung von Poly-Gamma-Glutamat beteiligtes Genprodukt codierende Gen *ywtB* funktionell inaktiviert ist, wobei die codierende Nukleotidsequenz *ywtB* eine Nukleotidsequenz besitzt, die zu der in SEQ ID NO. 7 angegebenen Nukleotidsequenz mindestens 94% Identität aufweist.

18. Mikroorganismus nach Anspruch 17, bei dem es sich um *Bacillus licheniformis* handelt.

## Claims

1. A method of reducing mucus attributable to poly-gamma-glutamate, wherein the protein YwtB (CapA, PgsA) encoded by the *ywtB* gene and having an amino acid sequence which is at least 90% identical to the amino acid sequence set forth in SEQ ID NO. 8 is prevented from functioning as an enzyme involved in the formation of polyamino acids or as a subunit of such an enzyme.

2. The method according to claim 1, wherein the YwtB protein is prevented from functioning during fermentation of the microorganism.

3. The method according to claim 1 or 2, **characterized by** a reduction in the mucus attributable to polyamino acids to 50%.

4. The method according to any of claims 1, 2 and 3, wherein the microorganism
a) is a bacterium, and/or
b) is a gram-negative bacterium, or
c) is a gram-positive bacterium.

5. The method according to claim 4, wherein the microorganism is selected from any of the genera *Escherichia, Klebsiella, Pseudomonas, Xanthomonas, Bacillus, Staphylococcus* and *Corynebacterium.*

6. The method according to any of the preceding claims, **characterized by** the following steps of preventing the YwtB protein encoded by the *ywtB* gene from functioning:
a) selecting two regions of the sequence SEQ ID NO. 7,
b) cloning said regions into a vector either to flank a section coding for an inactive protein or to follow directly upon each other, excluding the intermediate region,
c) deleting the *ywtB* gene using the vector produced in step b), and
d) detecting the gene deletion.

7. The method according to any of the preceding claims, wherein the YwtB (CapA, PsgA) protein encoded by the *ywtB* gene is prevented from functioning by employing a nucleic acid having a deletion or insertion mutation, preferably comprising the border sequences, each of which comprises at least 70 to 150 nucleic acid positions of the region coding for the protein.

8. A method of producing a valuable product by fermentation of a microorganism, wherein the formation of poly-gamma-glutamate by the microorganism during fermentation is reduced by preventing the YwtB (CapA, PgsA) protein encoded by the *ywtB* gene and having an amino acid sequence which is at least 90% identical to the amino acid sequence set forth in SEQ ID NO. 8 from functioning as an enzyme involved in the formation of polyamino acids or as a subunit of such an enzyme.

9. The method according to claim 8, wherein the YwtB (CapA, PgsA) protein encoded by the *ywtB* gene is prevented from functioning by a method comprising the following steps:
a) selecting two regions of the sequence SEQ ID NO. 7,
b) cloning said regions into a vector either to flank a section coding for an inactive protein or to follow directly upon each other, excluding the intermediate region,
c) deleting the *ywtB* gene using the vector produced in step b), and
d) detecting the gene deletion.

10. The method according to claim 8 or 9, wherein the YwtB (CapA, PsgA) protein encoded by the *ywtB* gene is prevented from functioning by employing a nucleic acid having a deletion or insertion mutation, preferably comprising the border sequences, each of which comprises at least 70 to 150 nucleic acid positions, of the region coding for the protein.

11. The method according to claim 8, 9 or 10, wherein the valuable product is a natural product, a food supplement or a pharmaceutically relevant compound or an enzyme.

12. The method according to claim 11, wherein the enzyme is selected from the group consisting of α-amylases, proteases, cellulases, lipases, oxidoreductases, peroxidases, laccases, oxidases, and hemicellulases.

13. The use of a nucleic acid coding for a YwtB (CapA, PgsA) protein involved in the formation of poly-gamma-glutamate and having an amino acid sequence which is at least 90% identical to the amino acid sequence set forth in SEQ ID NO. 8, or in each case parts thereof for preventing the YwtB (CapA, PgsA) protein from functioning as an enzyme involved in the formation of polyamino acids or as a subunit of such an enzyme.

14. The use of a nucleic acid coding for a YwtB (CapA, PgsA) protein involved in the formation of poly-gamma-glutamate and having an amino acid sequence which is at least 90% identical to the amino acid sequence set forth in SEQ ID NO. 8, or in each case parts thereof for reducing mucus attributable to poly-gamma-glutamate to 50% during fermentation of a microorganism.

15. The use according to claim 13 or 14, wherein the nucleic acid has the sequence SEQ ID NO. 7.

16. The use of a nucleic acid having a deletion or insertion mutation comprising the border sequences, each of which comprises at least 70 to 150 nucleic acid positions, of the region coding for a YwtB (CapA, PgsA) protein having an amino acid sequence which is at least 90% identical to the amino acid sequence set forth in SEQ ID NO. 8, for reducing mucus attributable to poly-gamma-glutamate during fermentation of a microorganism.

17. A microorganism in which the *ywtB* gene coding for a gene product involved in the formation of poly-gamma-glutamate has been functionally inactivated, the *ywtB* coding nucleotide sequence having a nucleotide sequence which is at least 94% identical to the nucleotide sequence set forth in SEQ ID NO. 7.

18. The microorganism according to claim 17, which is *Bacillus licheniformis.*

## Revendications

1. Procédé pour diminuer le mucilage dû au poly-gamma-glutamate, **caractérisé par** l'empêchement de la fonction de la protéine YwtB (CapA, PgsA) codée par le gène *ywtB* avec une séquence d'acides aminés qui a une identité d'au moins 90 % avec la séquence d'acides aminés indiquée dans SEQ ID NO:8, en tant qu'enzyme participant à la formation de poly(acides aminés), ou en tant que sous-unité d'une telle enzyme.

2. Procédé selon la revendication 1, **caractérisé en ce que** la fonction de la protéine YwtB est empêchée pendant la fermentation du microorganisme.

3. Procédé selon la revendication 1 ou 2, **caractérisé par** une diminution à 50 % du mucilage dû à des poly(acides aminés).

4. Procédé selon l'une des revendications 1, 2 ou 3, **caractérisé en ce que** le microorganisme
a) est une bactérie, et/ou
b) est une bactérie gram-négative, ou
c) est une bactérie gram-positive.

5. Procédé selon la revendication 4, **caractérisé en ce que** le microorganisme est choisi parmi l'un des genres *Escherichia, Klebsiella, Pseudomonas, Xanthomonas, Bacillus, Staphylococcus* ou *Corynebacterium.*

6. Procédé selon l'une des revendications précédentes, **caractérisé par** les étapes suivantes destinées à empêcher la fonction de la protéine *ywtB* codée par le gène YwtB :
a) sélection de deux domaines de la séquence SEQ ID NO:7,
b) clonage des domaines dans un vecteur de telle sorte qu'ils flanquent une partie codant pour une protéine non active, ou de telle sorte qu'ils se suivent directement, en négligeant le domaine intermédiaire,
c) délétion du gène *ywtB* avec le vecteur fabriqué dans l'étape b), et
d) détection de la délétion du gène.

7. Procédé selon l'une des revendications précédentes, **caractérisé en ce que** la fonction de la protéine YwtB (CapA, PsgA) codée par le gène *ywtB* est empêchée par l'utilisation d'un acide nucléique ayant une mutation par délétion ou par insertion, comprenant les séquences bordures, comprenant chacune au moins 70 à 150 positions d'acides nucléiques, du domaine codant pour la protéine.

8. Procédé de fabrication d'une substance utile par fermentation d'un microorganisme, **caractérisé en ce que**, pendant la fermentation, la formation de poly-gamma-glutamate par le microorganisme est diminuée par empêchement de la fonction de la protéine YwtB (CapA, PgsA) codée par le gène *ywtB* avec une séquence d'acides aminés qui présente une identité d'au moins 90 % avec la séquence d'acides aminés indiquée dans SEQ ID NO:8, en tant qu'enzyme participant à la formation de poly(acides aminés), ou en tant que sous-unité d'une telle enzyme.

9. Procédé selon la revendication 8, **caractérisé en ce que** la fonction de la protéine YwtB (CapA, PgsA) codée par le gène *ywtB* est empêchée par un procédé comportant les étapes suivantes :
a) sélection de deux domaines de la séquence SEQ ID NO:7,
b) clonage des domaines dans un vecteur de telle sorte qu'ils flanquent une partie codant pour une protéine non active, ou de telle sorte qu'ils se suivent directement, en négligeant le domaine intermédiaire,
c) délétion du gène *ywtB* avec le vecteur fabriqué dans l'étape b), et
d) détection de la délétion du gène.

10. Procédé selon la revendication 8 ou 9, **caractérisé en ce que** la fonction de la protéine YwtB (CapA, PsgA) codée par le gène *ywtB* est empêchée par l'utilisation d'un acide nucléique comportant une mutation par délétion ou par insertion, comprenant de préférence les séquences bordures, comprenant chacune au moins 70 à 150 positions d'acides nucléiques, du domaine codant pour la protéine.

11. Procédé selon la revendication 8, 9 ou 10, **caractérisé en ce que** la substance utile est une substance naturelle, un complément alimentaire ou un composé pharmaceutiquement pertinent, ou une enzyme.

12. Procédé selon la revendication 11, **caractérisé en ce que** l'enzyme est choisie dans le groupe des α-amylases, des protéases, des cellulases, des lipases, des oxydoréductases, des peroxydases, des laccases, des oxydases et des hémicellulases.

13. Utilisation d'un acide nucléique codant pour une protéine YwtB (CapA, PgsA) participant à la formation de poly-gamma-glutamate, ayant une séquence d'acides aminés qui présente une identité d'au moins 90 % avec la séquence d'acides aminés indiquée dans SEQ ID NO:8, ou des parties de celle-là, pour empêcher la fonction de la protéine YwtB (CapA, PgsA), en tant qu'enzyme participant à la formation de poly(acides aminés) ou en tant que sous-unité d'une telle enzyme.

14. Utilisation d'un acide nucléique codant pour une protéine YwtB (CapA, PgsA) participant à la formation de poly-gamma-glutamate, ayant une séquence d'acides aminés qui présente une identité d'au moins 90 % avec la séquence d'acides aminés indiquée dans SEQ ID NO:8, ou des parties de celle-là, pour diminuer à 50 %, pendant la fermentation d'un microorganisme, le mucilage dû au poly-gamma-glutamate.

15. Utilisation selon la revendication 13 ou 14, **caractérisée en ce que** l'acide nucléique a la séquence SEQ ID NO:7.

16. Utilisation d'un acide nucléique comportant une mutation par délétion ou par insertion, comprenant les séquences bordures, comprenant chacune au moins 70 à 150 positions d'acides nucléiques, du domaine codant pour une protéine YwtB (CapA, PsgA) ayant la séquence d'acides aminés qui présente une identité d'au moins 90 % avec la séquence d'acides aminés indiquée dans SEQ ID NO:8, pour diminuer le mucilage dû au poly-gamma-glutamate pendant la fermentation d'un microorganisme.

17. Microorganisme dans lequel le gène *ywtB* codant pour un produit génique participant à la formation du poly-gamma-glutamate est fonctionnellement inactivé, la séquence nucléotidique codante *ywtB* possédant une séquence nucléotidique qui présente une identité d'au moins 94 % avec la séquence nucléotidique indiquée dans SEQ ID NO:7.

18. Microorganisme selon la revendication 17, pour ce qui concerne lequel il s'agit de *Bacillus licheniformis.*


### SEQUENCE LISTING

<110> Henkel Kommanditgesellschaft auf Aktien
<120> Neue, Polyaminosäuren bildende oder abbauende Genprodukte von Bacillus licheniformis und darauf aufbauende verbesserte biotechnologische Produktionsverfahren
<130> H 06382 PCT
<150> DE102004030938.8
   <151> 2004-06-26
<160> 10
<170> PatentIn version 3.1
<210> 1
   <211> 1230
   <212> DNA
   <213> Bacillus licheniformis DSM 13
<220>
   <221> gene
   <222> (1) .. (1230)
   <223> ywsC
<220>
   <221> CDS
   <222> (1) .. (1230)
   <223>
<400> 1
   atg aat gaa ttt aca tat cag att cca aga agg aga tgt aga caa aca      48
   Met Asn Glu Phe Thr Tyr Gln Ile Pro Arg Arg Arg Cys Arg Gln Thr
   1               5                   10                  15
   atg tgg gta atg cta tta gcc tgt gtg atc gtt gtt ggg atc ggc att      96
   Met Trp Val Met Leu Leu Ala Cys Val Ile Val Val Gly Ile Gly Ile
               20                  25                  30
   tat gaa aaa agg cgc cac cag caa aat atc gat gcg ctg cct gtc cga      144
   Tyr Glu Lys Arg Arg His Gln Gln Asn Ile Asp Ala Leu Pro Val Arg
           35                  40                  45
   gtg aac atc aac ggt ata cgc gga aag tcc acg gtg aca aga tta aca      192
   Val Asn Ile Asn Gly Ile Arg Gly Lys Ser Thr Val Thr Arg Leu Thr
       50                  55                  60
   aca ggg ata tta atc gaa gca ggc tac aaa aca gta gga aaa aca acc      240
   Thr Gly Ile Leu Ile Glu Ala Gly Tyr Lys Thr Val Gly Lys Thr Thr
   65                  70                  75                  80
   ggg aca gac gca agg atg att tat tgg gac aca ccg gaa gag aag ccg      288
   Gly Thr Asp Ala Arg Met Ile Tyr Trp Asp Thr Pro Glu Glu Lys Pro
                   85                  90                  95
   atc aaa aga aag ccg caa ggg ccg aat atc gga gag cag aag gag gtt      336
   Ile Lys Arg Lys Pro Gln Gly Pro Asn Ile Gly Glu Gln Lys Glu Val
               100                 105                 110
   atg aaa gaa acg gtg gaa aga ggg gcc aat gcg att gtc agt gag tgc      384
   Met Lys Glu Thr Val Glu Arg Gly Ala Asn Ala Ile Val Ser Glu Cys
           115                 120                 125
   atg gcc gtt aat cct gat tac caa atc atc ttt cag gaa gaa ttg ctt      432
   Met Ala Val Asn Pro Asp Tyr Gln Ile Ile Phe Gln Glu Glu Leu Leu
       130                 135                 140
   cag gct aat atc ggc gtg atc gtg aac gtg ctg gag gat cac atg gat      480
   Gln Ala Asn Ile Gly Val Ile Val Asn Val Leu Glu Asp His Met Asp
   145                 150                 155                 160
   gtg atg gga ccg act ttg gat gaa atc gca gaa gca ttc aca gca acc      528
   Val Met Gly Pro Thr Leu Asp Glu Ile Ala Glu Ala Phe Thr Ala Thr
                   165                 170                 175
   att cct tat aat gga cat ttg gtt att act gat agt gag tat acc gat      576
   Ile Pro Tyr Asn Gly His Leu Val Ile Thr Asp Ser Glu Tyr Thr Asp
               180                 185                 190
   ttc ttt aag caa att gca aaa gaa agg aac aca aaa gtc atc gtc gca      624
   Phe Phe Lys Gln Ile Ala Lys Glu Arg Asn Thr Lys Val Ile Val Ala
           195                 200                 205
   gac aat tct aaa ata aca gat gaa tac ctc aga cag ttt gag tac atg      672
   Asp Asn Ser Lys Ile Thr Asp Glu Tyr Leu Arg Gln Phe Glu Tyr Met
       210                 215                 220
   gta ttc cct gat aat gcg tct ctt gcg ctc ggt gta gct caa gcg ttg      720
   Val Phe Pro Asp Asn Ala Ser Leu Ala Leu Gly Val Ala Gln Ala Leu
   225                 230                 235                 240
   ggc att gac gaa gaa acc gcc ttt aaa ggc atg ctg aat gcg ccg cct      768
   Gly Ile Asp Glu Glu Thr Ala Phe Lys Gly Met Leu Asn Ala Pro Pro
                   245                 250                 255
   gat ccg gga gcc atg aga att ctg ccg ctg atg aac gcc aag aat ccc      816
   Asp Pro Gly Ala Met Arg Ile Leu Pro Leu Met Asn Ala Lys Asn Pro
               260                 265                 270
   gga cat ttc gtc aac ggt ttt gcg gcc aat gac gca gct tcc act tta      864
   Gly His Phe Val Asn Gly Phe Ala Ala Asn Asp Ala Ala Ser Thr Leu
           275                 280                 285
   aac att tgg aag cgt gta aaa gaa ata ggc tat cct acg gat cag ccg      912
   Asn Ile Trp Lys Arg Val Lys Glu Ile Gly Tyr Pro Thr Asp Gln Pro
       290                 295                 300
   atc gtc att atg aac tgc cgc gcc gac agg gta gac aga aca cag cag      960
   Ile Val Ile Met Asn Cys Arg Ala Asp Arg Val Asp Arg Thr Gln Gln
   305                 310                 315                 320
   ttt gcg gaa gat gtc ctt cct tat att gaa gca agt gaa ctt gtg ctg     1008
   Phe Ala Glu Asp Val Leu Pro Tyr Ile Glu Ala Ser Glu Leu Val Leu
                   325                 330                 335
   att gga gaa aca aca gag ccg atc gtc aaa gca tat gaa gca ggc aaa      1056
   Ile Gly Glu Thr Thr Glu Pro Ile Val Lys Ala Tyr Glu Ala Gly Lys
               340                 345                 350
   att cct gcg gac aag ctg ttt gat ttt gag cac aaa tca acg gaa gaa      1104
   Ile Pro Ala Asp Lys Leu Phe Asp Phe Glu His Lys Ser Thr Glu Glu
           355                 360                 365
   atc atg ttc atg ctg aaa aac aag ctt gag ggc cgc gtt att tac gga      1152
   Ile Met Phe Met Leu Lys Asn Lys Leu Glu Gly Arg Val Ile Tyr Gly
       370                 375                 380
   gtc gga aat atc cac gga gca gcg gag cct ctc att gaa aaa ata caa      1200
   Val Gly Asn Ile His Gly Ala Ala Glu Pro Leu Ile Glu Lys Ile Gln
   385                 390                 395                 400
   gat tac aag att aag cag ctc gtt agc tag                              1230
   Asp Tyr Lys Ile Lys Gln Leu Val Ser
                   405
<210> 2
   <211> 409
   <212> PRT
   <213> Bacillus licheniformis DSM 13
<400> 2
   Met Asn Glu Phe Thr Tyr Gln Ile Pro Arg Arg Arg Cys Arg Gln Thr
   1               5                   10                  15
   Met Trp Val Met Leu Leu Ala Cys Val Ile Val Val Gly Ile Gly Ile
               20                  25                  30
   Tyr Glu Lys Arg Arg His Gln Gln Asn Ile Asp Ala Leu Pro Val Arg
           35                  40                  45
   Val Asn Ile Asn Gly Ile Arg Gly Lys Ser Thr Val Thr Arg Leu Thr
       50                  55                  60
   Thr Gly Ile Leu Ile Glu Ala Gly Tyr Lys Thr Val Gly Lys Thr Thr
   65                  70                  75                  80
   Gly Thr Asp Ala Arg Met Ile Tyr Trp Asp Thr Pro Glu Glu Lys Pro
                   85                  90                  95
   Ile Lys Arg Lys Pro Gln Gly Pro Asn Ile Gly Glu Gln Lys Glu Val
               100                 105                 110
   Met Lys Glu Thr Val Glu Arg Gly Ala Asn Ala Ile Val Ser Glu Cys
           115                 120                 125
   Met Ala Val Asn Pro Asp Tyr Gln Ile Ile Phe Gln Glu Glu Leu Leu
       130                 135                 140
   Gln Ala Asn Ile Gly Val Ile Val Asn Val Leu Glu Asp His Met Asp
   145                 150                 155                 160
   Val Met Gly Pro Thr Leu Asp Glu Ile Ala Glu Ala Phe Thr Ala Thr
                   165                 170                 175
   Ile Pro Tyr Asn Gly His Leu Val Ile Thr Asp Ser Glu Tyr Thr Asp
               180                 185                 190
   Phe Phe Lys Gln Ile Ala Lys Glu Arg Asn Thr Lys Val Ile Val Ala
           195                 200                 205
   Asp Asn Ser Lys Ile Thr Asp Glu Tyr Leu Arg Gln Phe Glu Tyr Met
       210                 215                 220
   Val Phe Pro Asp Asn Ala Ser Leu Ala Leu Gly Val Ala Gln Ala Leu
   225                 230                 235                 240
   Gly Ile Asp Glu Glu Thr Ala Phe Lys Gly Met Leu Asn Ala Pro Pro
                   245                 250                 255
   Asp Pro Gly Ala Met Arg Ile Leu Pro Leu Met Asn Ala Lys Asn Pro
               260                 265                 270
   Gly His Phe Val Asn Gly Phe Ala Ala Asn Asp Ala Ala Ser Thr Leu
           275                 280                 285
   Asn Ile Trp Lys Arg Val Lys Glu Ile Gly Tyr Pro Thr Asp Gln Pro
       290                 295                 300
   Ile Val Ile Met Asn Cys Arg Ala Asp Arg Val Asp Arg Thr Gln Gln
   305                 310                 315                 320
   Phe Ala Glu Asp Val Leu Pro Tyr Ile Glu Ala Ser Glu Leu Val Leu
                   325                 330                 335
   Ile Gly Glu Thr Thr Glu Pro Ile Val Lys Ala Tyr Glu Ala Gly Lys
               340                 345                 350
   Ile Pro Ala Asp Lys Leu Phe Asp Phe Glu His Lys Ser Thr Glu Glu
   355 360 365
   Ile Met Phe Met Leu Lys Asn Lys Leu Glu Gly Arg Val Ile Tyr Gly
   370 375 380
   Val Gly Asn Ile His Gly Ala Ala Glu Pro Leu Ile Glu Lys Ile Gln
   385 390 395 400
   Asp Tyr Lys Ile Lys Gln Leu Val Ser
   405
<210> 3
   <211> 1182
   <212> DNA
   <213> Bacillus licheniformis DSM 13
<220>
   <221> gene
   <222> (1)..(1182)
   <223> ywsC'
<220>
   <221> CDS
   <222> (1) .. (1182)
   <223>
<400> 3
   atg tgg gta atg cta tta gcc tgt gtg atc gtt gtt ggg atc ggc att 48
   Met Trp Val Met Leu Leu Ala Cys Val Ile Val Val Gly Ile Gly Ile
   1 5 10 15
   tat gaa aaa agg cgc cac cag caa aat atc gat gcg ctg cct gtc cga 96
   Tyr Glu Lys Arg Arg His Gln Gln Asn Ile Asp Ala Leu Pro Val Arg
   20 25 30
   gtg aac atc aac ggt ata cgc gga aag tcc acg gtg aca aga tta aca 144
   Val Asn Ile Asn Gly Ile Arg Gly Lys Ser Thr Val Thr Arg Leu Thr
   35 40 45
   aca ggg ata tta atc gaa gca ggc tac aaa aca gta gga aaa aca acc 192
   Thr Gly Ile Leu Ile Glu Ala Gly Tyr Lys Thr Val Gly Lys Thr Thr
   50 55 60
   ggg aca gac gca agg atg att tat tgg gac aca ccg gaa gag aag ccg 240
   Gly Thr Asp Ala Arg Met Ile Tyr Trp Asp Thr Pro Glu Glu Lys Pro
   65 70 75 80
   atc aaa aga aag ccg caa ggg ccg aat atc gga gag cag aag gag gtt 288
   Ile Lys Arg Lys Pro Gln Gly Pro Asn Ile Gly Glu Gln Lys Glu Val
   85 90 95
   atg aaa gaa acg gtg gaa aga ggg gcc aat gcg att gtc agt gag tgc 336
   Met Lys Glu Thr Val Glu Arg Gly Ala Asn Ala Ile Val Ser Glu Cys
               100                 105                 110
   atg gcc gtt aat cct gat tac caa atc atc ttt cag gaa gaa ttg ctt      384
   Met Ala Val Asn Pro Asp Tyr Gln Ile Ile Phe Gln Glu Glu Leu Leu
           115                 120                 125
   cag gct aat atc ggc gtg atc gtg aac gtg ctg gag gat cac atg gat      432
   Gln Ala Asn Ile Gly Val Ile Val Asn Val Leu Glu Asp His Met Asp
       130                 135                 140
   gtg atg gga ccg act ttg gat gaa atc gca gaa gca ttc aca gca acc      480
   Val Met Gly Pro Thr Leu Asp Glu Ile Ala Glu Ala Phe Thr Ala Thr
   145                 150                 155                 160
   att cct tat aat gga cat ttg gtt att act gat agt gag tat acc gat      528
   Ile Pro Tyr Asn Gly His Leu Val Ile Thr Asp Ser Glu Tyr Thr Asp
                   165                 170                 175
   ttc ttt aag caa att gca aaa gaa agg aac aca aaa gtc atc gtc gca      576
   Phe Phe Lys Gln Ile Ala Lys Glu Arg Asn Thr Lys Val Ile Val Ala
               180                 185                 190
   gac aat tct aaa ata aca gat gaa tac ctc aga cag ttt gag tac atg      624
   Asp Asn Ser Lys Ile Thr Asp Glu Tyr Leu Arg Gln Phe Glu Tyr Met
           195                 200                 205
   gta ttc cct gat aat gcg tct ctt gcg ctc ggt gta gct caa gcg ttg      672
   Val Phe Pro Asp Asn Ala Ser Leu Ala Leu Gly Val Ala Gln Ala Leu
       210                 215                 220
   ggc att gac gaa gaa acc gcc ttt aaa ggc atg ctg aat gcg ccg cct      720
   Gly Ile Asp Glu Glu Thr Ala Phe Lys Gly Met Leu Asn Ala Pro Pro
   225                 230                 235                 240
   gat ccg gga gcc atg aga att ctg ccg ctg atg aac gcc aag aat ccc      768
   Asp Pro Gly Ala Met Arg Ile Leu Pro Leu Met Asn Ala Lys Asn Pro
                   245                 250                 255
   gga cat ttc gtc aac ggt ttt gcg gcc aat gac gca gct tcc act tta      816
   Gly His Phe Val Asn Gly Phe Ala Ala Asn Asp Ala Ala Ser Thr Leu
               260                 265                 270
   aac att tgg aag cgt gta aaa gaa ata ggc tat cct acg gat cag ccg      864
   Asn Ile Trp Lys Arg Val Lys Glu Ile Gly Tyr Pro Thr Asp Gln Pro
           275                 280                 285
   atc gtc att atg aac tgc cgc gcc gac agg gta gac aga aca cag cag      912
   Ile Val Ile Met Asn Cys Arg Ala Asp Arg Val Asp Arg Thr Gln Gln
       290                 295                 300
   ttt gcg gaa gat gtc ctt cct tat att gaa gca agt gaa ctt gtg ctg      960
   Phe Ala Glu Asp Val Leu Pro Tyr Ile Glu Ala Ser Glu Leu Val Leu
   305                 310                 315                 320
   att gga gaa aca aca gag ccg atc gtc aaa gca tat gaa gca ggc aaa     1008
   Ile Gly Glu Thr Thr Glu Pro Ile Val Lys Ala Tyr Glu Ala Gly Lys
                   325                 330                 335
   att cct gcg gac aag ctg ttt gat ttt gag cac aaa tca acg gaa gaa     1056
   Ile Pro Ala Asp Lys Leu Phe Asp Phe Glu His Lys Ser Thr Glu Glu
               340                 345                 350
   atc atg ttc atg ctg aaa aac aag ctt gag ggc cgc gtt att tac gga      1104
   Ile Met Phe Met Leu Lys Asn Lys Leu Glu Gly Arg Val Ile Tyr Gly
           355                 360                 365
   gtc gga aat atc cac gga gca gcg gag cct ctc att gaa aaa ata caa      1152
   Val Gly Asn Ile His Gly Ala Ala Glu Pro Leu Ile Glu Lys Ile Gln
       370                 375                 380
   gat tac aag att aag cag ctc gtt agc tag                              1182
   Asp Tyr Lys Ile Lys Gln Leu Val Ser
   385                 390
<210> 4
   <211> 393
   <212> PRT
   <213> Bacillus licheniformis DSM 13
<400> 4
   Met Trp Val Met Leu Leu Ala Cys Val Ile Val Val Gly Ile Gly Ile
   1               5                   10                  15
   Tyr Glu Lys Arg Arg His Gln Gln Asn Ile Asp Ala Leu Pro Val Arg
               20                  25                  30
   Val Asn Ile Asn Gly Ile Arg Gly Lys Ser Thr Val Thr Arg Leu Thr
           35                  40                  45
   Thr Gly Ile Leu Ile Glu Ala Gly Tyr Lys Thr Val Gly Lys Thr Thr
       50                  55                  60
   Gly Thr Asp Ala Arg Met Ile Tyr Trp Asp Thr Pro Glu Glu Lys Pro
   65                  70                  75                  80
   Ile Lys Arg Lys Pro Gln Gly Pro Asn Ile Gly Glu Gln Lys Glu Val
                   85                  90                  95
   Met Lys Glu Thr Val Glu Arg Gly Ala Asn Ala Ile Val Ser Glu Cys
               100                 105                 110
   Met Ala Val Asn Pro Asp Tyr Gln Ile Ile Phe Gln Glu Glu Leu Leu
           115                 120                 125
   Gln Ala Asn Ile Gly Val Ile Val Asn Val Leu Glu Asp His Met Asp
       130                 135                 140
   Val Met Gly Pro Thr Leu Asp Glu Ile Ala Glu Ala Phe Thr Ala Thr
   145 150 155 160
   Ile Pro Tyr Asn Gly His Leu Val Ile Thr Asp Ser Glu Tyr Thr Asp
   165 170 175
   Phe Phe Lys Gln Ile Ala Lys Glu Arg Asn Thr Lys Val Ile Val Ala
   180 185 190
   Asp Asn Ser Lys Ile Thr Asp Glu Tyr Leu Arg Gln Phe Glu Tyr Met
   195 200 205
   Val Phe Pro Asp Asn Ala Ser Leu Ala Leu Gly Val Ala Gln Ala Leu
   210 215 220
   Gly Ile Asp Glu Glu Thr Ala Phe Lys Gly Met Leu Asn Ala Pro Pro
   225 230 235 240
   Asp Pro Gly Ala Met Arg Ile Leu Pro Leu Met Asn Ala Lys Asn Pro
   245 250 255
   Gly His Phe Val Asn Gly Phe Ala Ala Asn Asp Ala Ala Ser Thr Leu
   260 265 270
   Asn Ile Trp Lys Arg Val Lys Glu Ile Gly Tyr Pro Thr Asp Gln Pro
   275 280 285
   Ile Val Ile Met Asn Cys Arg Ala Asp Arg Val Asp Arg Thr Gln Gln
   290 295 300
   Phe Ala Glu Asp Val Leu Pro Tyr Ile Glu Ala Ser Glu Leu Val Leu
   305 310 315 320
   Ile Gly Glu Thr Thr Glu Pro Ile Val Lys Ala Tyr Glu Ala Gly Lys
   325 330 335
   Ile Pro Ala Asp Lys Leu Phe Asp Phe Glu His Lys Ser Thr Glu Glu
   340 345 350
   Ile Met Phe Met Leu Lys Asn Lys Leu Glu Gly Arg Val Ile Tyr Gly
   355 360 365
   Val Gly Asn Ile His Gly Ala Ala Glu Pro Leu Ile Glu Lys Ile Gln
   370 375 380
   Asp Tyr Lys Ile Lys Gln Leu Val Ser
   385 390
<210> 5
   <211> 450
   <212> DNA
   <213> Bacillus licheniformis DSM 13
<220>
   <221> gene
   <222> (1) .. (450)
   <223> ywtA
<220>
   <221> CDS
   <222> (1) .. (450)
   <223>
<400> 5
   atg ttt gga tca gat tta tat atc gcc ctc att tta gga gtc tta ctc       48
   Met Phe Gly Ser Asp Leu Tyr Ile Ala Leu Ile Leu Gly Val Leu Leu
   1               5                   10                  15
   agt ttg att ttt gca gag aaa acg gga att gta cca gcc ggc ctc gtc       96
   Ser Leu Ile Phe Ala Glu Lys Thr Gly Ile Val Pro Ala Gly Leu Val
               20                  25                  30
   gta ccg ggt tat ttg gga ctt gtc ttc aat cag ccg att ttc atg ctg      144
   Val Pro Gly Tyr Leu Gly Leu Val Phe Asn Gln Pro Ile Phe Met Leu
           35                  40                  45
   ctc gtt ctt ttt gtc agt ttg ctg acg tat gtc atc gtg aaa ttc gga      192
   Leu Val Leu Phe Val Ser Leu Leu Thr Tyr Val Ile Val Lys Phe Gly
       50                  55                  60
   ctt tcc aaa att atg att cta tac gga cgc aga aaa ttc gca gca atg      240
   Leu Ser Lys Ile Met Ile Leu Tyr Gly Arg Arg Lys Phe Ala Ala Met
   65                  70                  75                  80
   ctg att acg gga att ctt ttg aaa atc ggt ttt gat ttt ata tat ccg      288
   Leu Ile Thr Gly Ile Leu Leu Lys Ile Gly Phe Asp Phe Ile Tyr Pro
                   85                  90                  95
   gtg atg ccg ttt gag att gcc gaa ttc agg gga atc gga atc atc gtg      336
   Val Met Pro Phe Glu Ile Ala Glu Phe Arg Gly Ile Gly Ile Ile Val
               100                 105                 110
   ccg ggg ctg atc gcc aat acc att caa aga cag gga tta acg att acg      384
   Pro Gly Leu Ile Ala Asn Thr Ile Gln Arg Gln Gly Leu Thr Ile Thr
           115                 120                 125
   ctt gga agt acg ctt tta ttg agc gga gca aca ttc gtc att atg tat      432
   Leu Gly Ser Thr Leu Leu Leu Ser Gly Ala Thr Phe Val Ile Met Tyr
       130                 135                 140
   gct tac tat cta atc taa                                              450
   Ala Tyr Tyr Leu Ile
   145
<210> 6
   <211> 149
   <212> PRT
   <213> Bacillus licheniformis DSM 13
<400> 6
   Met Phe Gly Ser Asp Leu Tyr Ile Ala Leu Ile Leu Gly Val Leu Leu
   1               5                   10                  15
   Ser Leu Ile Phe Ala Glu Lys Thr Gly Ile Val Pro Ala Gly Leu Val
               20                  25                  30
   Val Pro Gly Tyr Leu Gly Leu Val Phe Asn Gln Pro Ile Phe Met Leu
           35                  40                  45
   Leu Val Leu Phe Val Ser Leu Leu Thr Tyr Val Ile Val Lys Phe Gly
       50                  55                  60
   Leu Ser Lys Ile Met Ile Leu Tyr Gly Arg Arg Lys Phe Ala Ala Met
   65                  70                  75                  80
   Leu Ile Thr Gly Ile Leu Leu Lys Ile Gly Phe Asp Phe Ile Tyr Pro
                   85                  90                  95
   Val Met Pro Phe Glu Ile Ala Glu Phe Arg Gly Ile Gly Ile Ile Val
               100                 105                 110
   Pro Gly Leu Ile Ala Asn Thr Ile Gln Arg Gln Gly Leu Thr Ile Thr
           115                 120                 125
   Leu Gly Ser Thr Leu Leu Leu Ser Gly Ala Thr Phe Val Ile Met Tyr
       130                 135                 140
   Ala Tyr Tyr Leu Ile
   145
<210> 7
   <211> 1170
   <212> DNA
   <213> Bacillus licheniformis DSM 13
<220>
   <221> gene
   <222> (1)..(1170)
   <223> ywtB
<220>
   <221> CDS
   <222> (1) .. (1170)
   <223>
<400> 7
   atg aaa aaa caa ctg aac ttt cag gaa aaa ctg ctg aag ttg acg aag      48
   Met Lys Lys Gln Leu Asn Phe Gln Glu Lys Leu Leu Lys Leu Thr Lys
   1               5                   10                  15
   cag gag aaa aag aaa aca aac aag cac gtc ttt atc gta ttg ccc gtt      96
   Gln Glu Lys Lys Lys Thr Asn Lys His Val Phe Ile Val Leu Pro Val
               20                  25                  30
   att ttc tgt tta atg ttt gtc ttt act tgg gtc gga agc gcc aaa act     144
   Ile Phe Cys Leu Met Phe Val Phe Thr Trp Val Gly Ser Ala Lys Thr
           35                  40                  45
   cct tcg caa atg gac aaa aaa gaa gat gcc aag ctt aca gct act ttt     192
   Pro Ser Gln Met Asp Lys Lys Glu Asp Ala Lys Leu Thr Ala Thr Phe
       50                  55                  60
   gtt ggc gat atc atg atg gga aga aac gta gaa aaa gtg aca aac ttg     240
   Val Gly Asp Ile Met Met Gly Arg Asn Val Glu Lys Val Thr Asn Leu
   65                  70                  75                  80
   cac ggt tcg gaa agt gtc ttc aaa aat gtg aag ccg tac ttt aat gtg     288
   His Gly Ser Glu Ser Val Phe Lys Asn Val Lys Pro Tyr Phe Asn Val
                   85                  90                  95
   tca gat ttt atc aca gga aac ttt gaa aac cct gta acc aat gca aag     336
   Ser Asp Phe Ile Thr Gly Asn Phe Glu Asn Pro Val Thr Asn Ala Lys
               100                 105                 110
   gac tat caa gag gca gaa aag aac atc cat ctg caa acg aat caa gaa     384
   Asp Tyr Gln Glu Ala Glu Lys Asn Ile His Leu Gln Thr Asn Gln Glu
           115                 120                 125
   tca gtc gaa aca ttg aaa aag ctg aac ttc agc gta ctg aat ttt gcc     432
   Ser Val Glu Thr Leu Lys Lys Leu Asn Phe Ser Val Leu Asn Phe Ala
       130                 135                 140
   aac aac cat gcg atg gac tac ggg gaa gac ggt ttg aag gat acg ctc     480
   Asn Asn His Ala Met Asp Tyr Gly Glu Asp Gly Leu Lys Asp Thr Leu
   145                 150                 155                 160
   aat aaa ttt tca aat gag aat ctg gag ctt gtc gga gca gga aat aat     528
   Asn Lys Phe Ser Asn Glu Asn Leu Glu Leu Val Gly Ala Gly Asn Asn
                   165                 170                 175
   ctt gaa gac gcg aaa cag cac gta tcc tat cag aat gtg aac ggc gta     576
   Leu Glu Asp Ala Lys Gln His Val Ser Tyr Gln Asn Val Asn Gly Val
               180                 185                 190
   aaa att gca acg ctc ggt ttt aca gac gtc tac aca aag aac ttt aca      624
   Lys Ile Ala Thr Leu Gly Phe Thr Asp Val Tyr Thr Lys Asn Phe Thr
           195                 200                 205
   gcc aaa aag aac aga ggc gga gtg ctg ccg ctc agt ccg aaa atc ttt      672
   Ala Lys Lys Asn Arg Gly Gly Val Leu Pro Leu Ser Pro Lys Ile Phe
       210                 215                 220
   att cca atg att gcg gaa gca tcg aaa aaa gcg gat ctt gtc ctt gtc      720
   Ile Pro Met Ile Ala Glu Ala Ser Lys Lys Ala Asp Leu Val Leu Val
   225                 230                 235                 240
   cat gtg cac tgg gga caa gaa tat gac aat gaa ccg aac gac aga cag      768
   His Val His Trp Gly Gln Glu Tyr Asp Asn Glu Pro Asn Asp Arg Gln
                   245                 250                 255
   aag gat ctg gcc aag gcg att gca gat gcc gga gca gat gtc atc atc      816
   Lys Asp Leu Ala Lys Ala Ile Ala Asp Ala Gly Ala Asp Val Ile Ile
               260                 265                 270
   ggc gct cat ccc cat gtt ctc gaa ccg atc gaa gtg tat aac ggt act      864
   Gly Ala His Pro His Val Leu Glu Pro Ile Glu Val Tyr Asn Gly Thr
           275                 280                 285
   gtg att ttc tac agc ctc ggc aac ttt gta ttt gat cag ggc tgg tca      912
   Val Ile Phe Tyr Ser Leu Gly Asn Phe Val Phe Asp Gln Gly Trp Ser
       290                 295                 300
   aga aca cgg gac agc gcg ctt gta caa tac cat tta atg aat gac ggc      960
   Arg Thr Arg Asp Ser Ala Leu Val Gln Tyr His Leu Met Asn Asp Gly
   305                 310                 315                 320
   aaa ggg cgc ttt gag gta acg cct ctc aac att cgc gaa gca acg ccg     1008
   Lys Gly Arg Phe Glu Val Thr Pro Leu Asn Ile Arg Glu Ala Thr Pro
                   325                 330                 335
   acg cct tta ggc aag agc gac ttc tta aaa cga aaa gcg atc ttc cgt     1056
   Thr Pro Leu Gly Lys Ser Asp Phe Leu Lys Arg Lys Ala Ile Phe Arg
               340                 345                 350
   caa ttg aca aaa gga aca aac ctc gac tgg aaa gaa gag aac gga aaa     1104
   Gln Leu Thr Lys Gly Thr Asn Leu Asp Trp Lys Glu Glu Asn Gly Lys
           355                 360                 365
   tta acg ttt gaa gtc gat cat gcg gac aag ctg aaa aat aat aaa aac     1152
   Leu Thr Phe Glu Val Asp His Ala Asp Lys Leu Lys Asn Asn Lys Asn
       370                 375                 380
   gga gtg gtg aac aaa tga                                             1170
   Gly Val Val Asn Lys
   385
<210> 8
   <211> 389
   <212> PRT
   <213> Bacillus licheniformis DSM 13
<400> 8
   Met Lys Lys Gln Leu Asn Phe Gln Glu Lys Leu Leu Lys Leu Thr Lys
   1               5                   10                  15
   Gln Glu Lys Lys Lys Thr Asn Lys His Val Phe Ile Val Leu Pro Val
               20                  25                  30
   Ile Phe Cys Leu Met Phe Val Phe Thr Trp Val Gly Ser Ala Lys Thr
           35                  40                  45
   Pro Ser Gln Met Asp Lys Lys Glu Asp Ala Lys Leu Thr Ala Thr Phe
       50                  55                  60
   Val Gly Asp Ile Met Met Gly Arg Asn Val Glu Lys Val Thr Asn Leu
   65                  70                  75                  80
   His Gly Ser Glu Ser Val Phe Lys Asn Val Lys Pro Tyr Phe Asn Val
                   85                  90                  95
   Ser Asp Phe Ile Thr Gly Asn Phe Glu Asn Pro Val Thr Asn Ala Lys
               100                 105                 110
   Asp Tyr Gln Glu Ala Glu Lys Asn Ile His Leu Gln Thr Asn Gln Glu
           115                 120                 125
   Ser Val Glu Thr Leu Lys Lys Leu Asn Phe Ser Val Leu Asn Phe Ala
       130                 135                 140
   Asn Asn His Ala Met Asp Tyr Gly Glu Asp Gly Leu Lys Asp Thr Leu
   145                 150                 155                 160
   Asn Lys Phe Ser Asn Glu Asn Leu Glu Leu Val Gly Ala Gly Asn Asn
                   165                 170                 175
   Leu Glu Asp Ala Lys Gln His Val Ser Tyr Gln Asn Val Asn Gly Val
               180                 185                 190
   Lys Ile Ala Thr Leu Gly Phe Thr Asp Val Tyr Thr Lys Asn Phe Thr
           195                 200                 205
   Ala Lys Lys Asn Arg Gly Gly Val Leu Pro Leu Ser Pro Lys Ile Phe
       210                 215                 220
   Ile Pro Met Ile Ala Glu Ala Ser Lys Lys Ala Asp Leu Val Leu Val
   225                 230                 235                 240
   His Val His Trp Gly Gln Glu Tyr Asp Asn Glu Pro Asn Asp Arg Gln
                   245                 250                 255
   Lys Asp Leu Ala Lys Ala Ile Ala Asp Ala Gly Ala Asp Val Ile Ile
               260                 265                 270
   Gly Ala His Pro His Val Leu Glu Pro Ile Glu Val Tyr Asn Gly Thr
           275                 280                 285
   Val Ile Phe Tyr Ser Leu Gly Asn Phe Val Phe Asp Gln Gly Trp Ser
       290                 295                 300
   Arg Thr Arg Asp Ser Ala Leu Val Gln Tyr His Leu Met Asn Asp Gly
   305                 310                 315                 320
   Lys Gly Arg Phe Glu Val Thr Pro Leu Asn Ile Arg Glu Ala Thr Pro
                   325                 330                 335
   Thr Pro Leu Gly Lys Ser Asp Phe Leu Lys Arg Lys Ala Ile Phe Arg
               340                 345                 350
   Gln Leu Thr Lys Gly Thr Asn Leu Asp Trp Lys Glu Glu Asn Gly Lys
           355                 360                 365
   Leu Thr Phe Glu Val Asp His Ala Asp Lys Leu Lys Asn Asn Lys Asn
       370                 375                 380
   Gly Val Val Asn Lys
   385
<210> 9
   <211> 1245
   <212> DNA
   <213> Bacillus licheniformis DSM 13
<220>
   <221> gene
   <222> (1)..(1245)
   <223> ywtD
<220>
   <221> CDS
   <222> (1) .. (1295)
   <223>
<220>
   <221> misc_feature
   <222> (1) .. (3)
   <223> First codon translated as Met.
<400> 9
   ttg ata aaa aaa gcg gca aac aaa aag ttg gtt ttg ttt tgt gga att      48
   Leu Ile Lys Lys Ala Ala Asn Lys Lys Leu Val Leu Phe Cys Gly Ile
   1               5                   10                  15
   gcg gtg ctt tgg atg tct tta ttt tta acg aat cat aat gat gta cgc      96
   Ala Val Leu Trp Met Ser Leu Phe Leu Thr Asn His Asn Asp Val Arg
               20                  25                  30
   gcc gat acg atc ggc gag aaa ata gcg gaa act gcc aga cag ctt gag     144
   Ala Asp Thr Ile Gly Glu Lys Ile Ala Glu Thr Ala Arg Gln Leu Glu
           35                  40                  45
   ggt gcg aaa tac agc tac ggc gga gag aag ccg aaa acg ggg ttt gac     192
   Gly Ala Lys Tyr Ser Tyr Gly Gly Glu Lys Pro Lys Thr Gly Phe Asp
       50                  55                  60
   tcg tca ggc ttt gtg caa tat gtg ttt caa tcg ctc gat att acg ctt     240
   Ser Ser Gly Phe Val Gln Tyr Val Phe Gln Ser Leu Asp Ile Thr Leu
   65                  70                  75                  80
   ccg aga acg gta aag gaa caa tcg act ctt ggg agc agt gtc ggc cgt     288
   Pro Arg Thr Val Lys Glu Gln Ser Thr Leu Gly Ser Ser Val Gly Arg
                   85                  90                  95
   cag cag ctc gaa aag ggg gac ctt gtc ttt ttc aag aat gcc gag ctg     336
   Gln Gln Leu Glu Lys Gly Asp Leu Val Phe Phe Lys Asn Ala Glu Leu
               100                 105                 110
   gaa tcg gac gga ccg acc cat gtc gcc atc tat ttg gga aat gat caa     384
   Glu Ser Asp Gly Pro Thr His Val Ala Ile Tyr Leu Gly Asn Asp Gln
           115                 120                 125
   atc atc cac agc aca aaa tca aac ggg gtt gtc gtg aca aag ctt gaa     432
   Ile Ile His Ser Thr Lys Ser Asn Gly Val Val Val Thr Lys Leu Glu
       130                 135                 140
   ggc agc tct tac tgg agc tcg ggg tat ttt aaa gcg aaa agg atc aca     480
   Gly Ser Ser Tyr Trp Ser Ser Gly Tyr Phe Lys Ala Lys Arg Ile Thr
   145                 150                 155                 160
   aaa gag cct gag att tcg atg gat cct gtc gtt caa aaa gca aaa agc     528
   Lys Glu Pro Glu Ile Ser Met Asp Pro Val Val Gln Lys Ala Lys Ser
                   165                 170                 175
   tat gtc ggt gtt cct tat gta ttt gga ggc aac tct ccg gat ctc gga     576
   Tyr Val Gly Val Pro Tyr Val Phe Gly Gly Asn Ser Pro Asp Leu Gly
               180                 185                 190
   ttt gac tgt tcg ggg ttg acc caa tac gtc ttc aga gag gtg ctc ggc     624
   Phe Asp Cys Ser Gly Leu Thr Gln Tyr Val Phe Arg Glu Val Leu Gly
           195                 200                 205
   gtt tat ttg cca agg tcg gct gaa cag caa tgg gct gtc ggt caa aag      672
   Val Tyr Leu Pro Arg Ser Ala Glu Gln Gln Trp Ala Val Gly Gln Lys
       210                 215                 220
   gtg aag ctt gaa gat atc cgg ccg ggt gat gtt ttg ttt ttc agc aat      720
   Val Lys Leu Glu Asp Ile Arg Pro Gly Asp Val Leu Phe Phe Ser Asn
   225                 230                 235                 240
   acg tac aaa ccg gga ata tcc cat aac ggc atc tat gcc ggg ggc ggg      768
   Thr Tyr Lys Pro Gly Ile Ser His Asn Gly Ile Tyr Ala Gly Gly Gly
                   245                 250                 255
   cgg ttt atc cat gcg agc cgt tca aat aaa gtg acg ata tcc tac ttg      816
   Arg Phe Ile His Ala Ser Arg Ser Asn Lys Val Thr Ile Ser Tyr Leu
               260                 265                 270
   tcg gct tcc tat tgg cag aag aag ttc aca gga gtc aga cgt ttt gac      864
   Ser Ala Ser Tyr Trp Gln Lys Lys Phe Thr Gly Val Arg Arg Phe Asp
           275                 280                 285
   aac atg tcc ctg cca aaa aat ccg att gta tcc gaa gcc atc agg cat      912
   Asn Met Ser Leu Pro Lys Asn Pro Ile Val Ser Glu Ala Ile Arg His
       290                 295                 300
   atc ggc gaa gtc ggt tat caa aaa ggc ggc aca tcg cct aaa gaa ggc      960
   Ile Gly Glu Val Gly Tyr Gln Lys Gly Gly Thr Ser Pro Lys Glu Gly
   305                 310                 315                 320
   ttt gat acg gct ggg ttt atc caa tat gtc tac aaa acg gcg gca gga     1008
   Phe Asp Thr Ala Gly Phe Ile Gln Tyr Val Tyr Lys Thr Ala Ala Gly
                   325                 330                 335
   gtg gag ctt ccg agg tat gct gac aaa caa tac agc acg ggt aag aaa     1056
   Val Glu Leu Pro Arg Tyr Ala Asp Lys Gln Tyr Ser Thr Gly Lys Lys
               340                 345                 350
   att acc aaa cag gag ctt gag cct gga gac atc gtc ttc ttt aaa gga     1104
   Ile Thr Lys Gln Glu Leu Glu Pro Gly Asp Ile Val Phe Phe Lys Gly
           355                 360                 365
   acc act gtt atg aat ccc gcc atc tat atc gga aac ggc cag gtc gtt     1152
   Thr Thr Val Met Asn Pro Ala Ile Tyr Ile Gly Asn Gly Gln Val Val
       370                 375                 380
   ctt gtc acc ttg tct gcc ggt gta acg aca gca gat atg gag acg agc     1200
   Leu Val Thr Leu Ser Ala Gly Val Thr Thr Ala Asp Met Glu Thr Ser
   385                 390                 395                 400
   gcc tat tgg aaa gat aaa tac gcc gga agc gtc aga att gag tag         1245
   Ala Tyr Trp Lys Asp Lys Tyr Ala Gly Ser Val Arg Ile Glu
                   405                 410
<210> 10
   <211> 414
   <212> PRT
   <213> Bacillus licheniformis DSM 13
<220>
   <221> misc_feature
   <222> (1) .. (3)
   <223> First codon translated as Met.
<400> 10
   Leu Ile Lys Lys Ala Ala Asn Lys Lys Leu Val Leu Phe Cys Gly Ile
   1               5                   10                  15
   Ala Val Leu Trp Met Ser Leu Phe Leu Thr Asn His Asn Asp Val Arg
               20                  25                  30
   Ala Asp Thr Ile Gly Glu Lys Ile Ala Glu Thr Ala Arg Gln Leu Glu
           35                  40                  45
   Gly Ala Lys Tyr Ser Tyr Gly Gly Glu Lys Pro Lys Thr Gly Phe Asp
       50                  55                  60
   Ser Ser Gly Phe Val Gln Tyr Val Phe Gln Ser Leu Asp Ile Thr Leu
   65                  70                  75                  80
   Pro Arg Thr Val Lys Glu Gln Ser Thr Leu Gly Ser Ser Val Gly Arg
                   85                  90                  95
   Gln Gln Leu Glu Lys Gly Asp Leu Val Phe Phe Lys Asn Ala Glu Leu
               100                 105                 110
   Glu Ser Asp Gly Pro Thr His Val Ala Ile Tyr Leu Gly Asn Asp Gln
           115                 120                 125
   Ile Ile His Ser Thr Lys Ser Asn Gly Val Val Val Thr Lys Leu Glu
       130                 135                 140
   Gly Ser Ser Tyr Trp Ser Ser Gly Tyr Phe Lys Ala Lys Arg Ile Thr
   145                 150                 155                 160
   Lys Glu Pro Glu Ile Ser Met Asp Pro Val Val Gln Lys Ala Lys Ser
                   165                 170                 175
   Tyr Val Gly Val Pro Tyr Val Phe Gly Gly Asn Ser Pro Asp Leu Gly
               180                 185                 190
   Phe Asp Cys Ser Gly Leu Thr Gln Tyr Val Phe Arg Glu Val Leu Gly
           195                 200                 205
   Val Tyr Leu Pro Arg Ser Ala Glu Gln Gln Trp Ala Val Gly Gln Lys
       210                 215                 220
   Val Lys Leu Glu Asp Ile Arg Pro Gly Asp Val Leu Phe Phe Ser Asn
   225                 230                 235                 240
   Thr Tyr Lys Pro Gly Ile Ser His Asn Gly Ile Tyr Ala Gly Gly Gly
                   245                 250                 255
   Arg Phe Ile His Ala Ser Arg Ser Asn Lys Val Thr Ile Ser Tyr Leu
               260                 265                 270
   Ser Ala Ser Tyr Trp Gln Lys Lys Phe Thr Gly Val Arg Arg Phe Asp
           275                 280                 285
   Asn Met Ser Leu Pro Lys Asn Pro Ile Val Ser Glu Ala Ile Arg His
       290                 295                 300
   Ile Gly Glu Val Gly Tyr Gln Lys Gly Gly Thr Ser Pro Lys Glu Gly
   305                 310                 315                 320
   Phe Asp Thr Ala Gly Phe Ile Gln Tyr Val Tyr Lys Thr Ala Ala Gly
                   325                 330                 335
   Val Glu Leu Pro Arg Tyr Ala Asp Lys Gln Tyr Ser Thr Gly Lys Lys
               340                 345                 350
   Ile Thr Lys Gln Glu Leu Glu Pro Gly Asp Ile Val Phe Phe Lys Gly
           355                 360                 365
   Thr Thr Val Met Asn Pro Ala Ile Tyr Ile Gly Asn Gly Gln Val Val
       370                 375                 380
   Leu Val Thr Leu Ser Ala Gly Val Thr Thr Ala Asp Met Glu Thr Ser
   385                 390                 395                 400
   Ala Tyr Trp Lys Asp Lys Tyr Ala Gly Ser Val Arg Ile Glu
                   405                 410